Europäisches Patentamt

European Patent Office

Office européen des brevets

(11) Publication number: **0 202 300 B1**

(12) **EUROPEAN PATENT SPECIFICATION**

(45) Date of publication of patent specification: **11.11.92**

(51) Int. Cl.⁵: **C12N 15/27**, C12P 21/02, C07H 21/04, A61K 37/02

(21) Application number: **85906001.4**

(22) Date of filing: **18.11.85**

(86) International application number:
**PCT/US85/02250**

(87) International publication number:
**WO 86/03225 (05.06.86 86/12)**

(54) **cDNA CLONES CODING FOR POLYPEPTIDES EXHIBITING HUMAN GRANULOCYTE MACROPHAGE AND EOSINOPHIL CELLULAR GROWTH FACTOR ACTIVITY.**

(30) Priority: **20.11.84 US 673898**

(43) Date of publication of application:
**26.11.86 Bulletin 86/48**

(45) Publication of the grant of the patent:
**11.11.92 Bulletin 92/46**

(84) Designated Contracting States:
**AT BE CH DE FR GB IT LI LU NL SE**

(56) References cited:
**EP-A- 0 183 350**
**WO-A-85/04188**
**US-A- 4 438 032**

**Proc. Natl. Acad. Sci (USA), vol. 82, September 1985. M. Cantrell et al "Cloning, sequence and expression of a human granulocyte-macrophage colony stimulating factor", pages 6250-6254**

(73) Proprietor: **Schering Biotech Corporation**
**901 California Avenue**
**Palo Alto California 94304-1104(US)**

(72) Inventor: **YOKOTA, Takashi**
**890 Colorado Avenue**
**Palo Alto, CA 94303(US)**
Inventor: **LEE, Frank, Don**
**212 Rinconada Avenue**
**Palo Alto, CA 94301(US)**
Inventor: **RENNICK, Donna, Maye**
**601 Almond Avenue**
**Los Altos, CA 94022(US)**
Inventor: **ARAI, Ken-ichi**
**648 Georgia Avenue**
**Palo Alto, CA 94306(US)**

(74) Representative: **Ritter, Stephen David et al**
**Mathys & Squire 10 Fleet Street**
**London EC4Y 1AY(GB)**

Chemical abstracts, vol. 103, 1985, Columbus, Ohio, USA. E.S. Kawasaski et al "Molecular cloning of a complementary DNA encoding human macrophage-specific colony-stimulating factor (CSF-1)", see page 269, abstract n 208048z & Science (Washington, D.C. 1883), 1985, 230(4723), 291-6 (Eng.)

Nature, vol. 307, 19 January 1984, M.C. Fung et al "Molecular cloning of cDNA for murine interleukin-3", pages 233-237

Proc. Natl. Acad. Sci. USA, vol. 81, February 1984, Takashi Yokota et al "Isolation and characterization of a mouse cDNA clone that expresses mast-cell growth-factor activity in monkey cells", pages 1070-1074

Sciences, Washington D.C. 1883) 1985, 228 (4701) Wong, Gordon G. et al "Human GM-CSF : molecular cloning of the complementary DNA and purification of the natural and recombinant proteins", pages 810-815

Proc. Natl. Acad. Sci. USA, vol. 82, July 1985. Frank Lee et al "Isolation of cDNA for a human granulocyte-macrophage colony-stimulating factor by functional expression in mammalion cells", pages 4360-4364

Nature, vol. 298, July 1982. Aldons J. Lusis et al "Translation of mRNA for human granulocyte-macrophage colony stimulating factor", pages 75-77

Nature, vol. 309, 28June 1984, Nicholas M. Gough et al "Molecular cloning of cDNA encoding a murine haematopoietic growth regulator, granulocyte-macrophage colony stimulating factor", pages 763-767

## Description

This invention relates generally to the application of recombinant DNA technology to elucidate the control mechanisms of the mammalian immune response and, more particularly, to the isolation of nucleic acid clones coding for polypeptides exhibiting human granulocyte/macrophage growth factor activity, including eosinophil growth factor activity.

Recombinant DNA technology refers generally to the technique of integrating genetic information from a donor source into vectors for subsequent processing, such as through introduction into a host, whereby the transferred genetic information is copied and/or expressed in the new environment. Commonly, the genetic information exists in the form of complementary DNA (cDNA) derived from messenger RNA (mRNA) coding for a desired protein product. The carrier is frequently a plasmid having the capacity to incorporate cDNA for later replication in a host and, in some cases, actually to control expression of the cDNA and thereby direct synthesis of the encoded product in the host.

This technology has progressed extremely rapidly in recent years and a variety of exogenous proteins have been expressed in a variety of hosts, but obtaining any desired novel cDNA clone remains an uncertainty. By way of example, some of the eukaryotic proteins produced by recombinant DNA technology include: proinsulin (Naber, S. et al., Gene 21: 95-104 [1983]); interferons (Simon, L. et al., Proc. Nat. Acad. Sci. U.S.A., 80: 2059-2062 [1983] and Derynck, R. et al., Nucl. Acids Res. 1: 1819-1837 [1983]); growth hormone (Goeddel, D., et al., Nature 281: 544-548 [1979]); and mast cell growth factor (Yokota, T. et al., Proc. Nat. Acad. Sci. U.S.A., 81: 1070-1074 [1984]). (These publications and other reference materials cited hereafter have been included to provide additional details on the background of the pertinent art and, in particular instances, the practice of invention, and are all incorporated herein by reference.)

For some time, it has been dogma that the mammalian immune response was due primarily to a series of complex cellular interactions, called the "immune network". While it remains clear that much of the response does in fact revolve around the network-like interactions of lymphocytes, macrophages, granulocytes and other cells, immunologists now generally hold the opinion that soluble proteins (e.g. the so-called lymphokines) play a critical role in controlling these cellular interactions.

Lymphokines apparently mediate cellular activities in a variety of ways. They have been shown to have the ability to support the proliferation and growth of various hematopoietic cells and, indeed, are thought to play a crucial role in the basic differentiation of pluripotential hematopoietic stem cells into the vast number of progenitors of the diverse cellular lineages responsible for the immune response. Cell lineages important in this response include two classes of lymphocytes: B cells, which can produce and secrete immunoglobulins (proteins with the capability of recognizing and binding to foreign matter to effect its removal), and T cells (of various subsets) that through various mechanisms induce or suppress B cells and some of the other cells (including other T cells) making up the immune network.

Another important white blood cell type is the phagocyte, particularly the polymorphonuclear and mononuclear leukocytes. These cells are thought to specialize in scavenging, a capability essential for assistance in the elimination of invading organisms and in the removal of defunct cells and extracellular debris.

Within these leukocyte cell types are the granulocytes, including the neutrophils and eosinophils. Neutrophils can be found in the peripheral blood, many tissues and most body areas having direct contact with the environment (e.g., oral cavity, bronchotracheal tree and cervical canal). Their phagocyte response to microbial invasion has been well studied (Klebanoff, S. and Clark, R., The Neutrophil: Function and Clinical Disorders, Elsevier/North Holland Biomedical Prep., Amsterdam [1978]), with the destruction of ingested organisms being accomplished primarily through the controlled release into vacuoles of various antimicrobial agents (e.g., oxygen radicals, $H_2O_2$ and halide ions). Much less is known about eosinophils, although they are readily identifiable by a very characteristic granulation pattern and have a documented association with allergic disorders and certain parasitic diseases, such as trichinosis.

Macrophages are dissimilar to granulocytes in that they develop distinct characteristics depending upon the surrounding tissue. Thus, tissue macrophages include histiocytes in connective tissue, Kupffer cells in the liver, alveolar macrophages in the lungs, osteoclasts in the bone, microbial cells in the nervous system, Langerhaus cells in the skin, as well as free or fixed cells in other organs. In addition to phagocytic ability, macrophages can secrete many very important biologically active substances, such as lysozyme, plasminogen activator, collagenase, elastase, acid hydrolases, complement components, prostaglandins, endogenous pyrogen, some lymphokines, and oxygen metabolites. Also, macrophages are believed to regulate the supply and presentation of antigens to B and T lymphocytes (Cline, M., The White Cell, Harvard University Press, Cambridge, Mass. [1975]).

Research to better understand (and thus potentially treat therapeutically) neutropenia, lymphopenia, monocytopenia, leukemia or a leukemoid reaction, as well as other immune disorders, through the study of macrophages, granulocytes, T cells, and the other cells involved in the immune response, has been hampered by the general inability to maintain these cells in vitro. However, several immunologists recently discovered that many such cells could be isolated and in some cases cultured by growing them on secretions from other cells, e.g., conditioned media from splenic lymphocytes stimulated with Concanavalin A (ConA). It has now become clear from this work that the generation of cell clones is dependent on specific factors, such as lymphokines.

Apparently almost all blood cell types are continuously generated in the adult vertebrate bone marrow through the growth and differentiation of the hierarchy of hematopoietic progenitor cells. At the apex of this hierarchy is the pluripotent stem cell, which can repopulate a lethally irradiated animal for most, if not all, hematological cell types (e.g., red cells, platelets, lymphocytes, various granulocytes and monocytes/macrophages). The pluripotent cell not only has the capacity to regenerate the pluripotent stem cell compartments (self-renewal), but also gives rise to progenitor cells committed to development along one particular lineage pathway. Progeny of a particular committed stem cell appear to share the same lineage commitment as the parent cell (Metcalf, D., Hemopoietic Colonies, Springer Publishing Co., New York, N.Y. [1977]).

In vitro studies on hematopoiesis have shown that a number of soluble factors can regulate the growth and differentiation of these various progenitor and committed cells. Some of these factors have been partially purified and shown to affect specifically stem cells belonging to a particular cell lineage. For example, erythropoietin, produced primarily in the kidneys, stimulates more-differentiated members of the erythroid hierarchy (Miyake, T., et al., J. Biol. Chem. 252: 5558 [1977]), and colony stimulating activity, produced in T-cells and macrophages, preferentially stimulates granulocytes and macrophage growth in semi-solid cultures of bone marrow cells (Stanley, E., and Heard, P., J. Biol. Chem. 252: 4305 [1977]). Yet another type of growth factor seems able to stimulate hematopoietic colonies consisting of single cell types and mixtures of cells. The range of cells, e.g. preerythrocytes, megakaryocytes, granulocytes, mast cells and monocyte/macrophages, that are apparently responsive to one factor of this second type have caused it to be named a multi-lineage cellular growth factor (Iscove, N. et al., J. Cell. Physiol. Suppl., 1: 65-78 [1982]-), indicating its capability of affecting a number of committed progenitor cells and pluripotential stem cells as well.

The colony-stimulating activity is known to exist in several molecular forms, which are also known collectively as colony stimulating factors (CSF's). CSF's are a family of glycoproteins with molecular weights ranging from about 20,000 to 70,000 daltons, and can be found circulating in the blood and in urine. The set of factors capable of stimulating both granulocyte and macrophage colony formation in semi-solid cultures (see Metcalf, D., Hematopoietic Colonies; In Vitro Cloning of Normal and Leukemic Cells; Springer Publishing Co., New York [1977]) is known as "GM-CSF."

Whereas both mouse and human GM-CSF's have been at least partially purified and characterized biochemically, vast discrepancies have been reported on both molecular weights and spectrum of activities - even intra-species (Metcalf, D., "Hematopoietic Colony Stimulating Factors" in Handbook of Experimental Pharmacology, 57: 343-384, Springer-Verlag, New York [1978]). The successful cloning of cDNA's encoding a murine GM-CSF activity (Gough, N. et al., Nature 309: 763-767 [1984]) should help answer many of the exant questions surrounding murine GM-CSF, but the problems remain in the human system. At least two groups of researchers have reported two human GM-CSF's (Das, S. et al., Blood 58: 630-641 [1981] and Nicola, N. et al., Blood 54: 614-627 [1979]). Indeed, even with the in vitro translation of mRNA (isolated from a T-lymphocyte cell line, ATCC accession number CRL8066; see U.S. patent no. 4,438,032) encoding human GM-CSF to yield reportedly biologically active protein (Lusis, A. et al., Nature 298: 75-77 [1982]), the uncertainty surrounding the human CSF's remains enormous (see, Burgess, A., Growth Factors and Stem Cells, Chapter 4, pgs. 93-124, Academic Press, New York [1984]).

Although molecular weight differences could perhaps be partially explained by varying amounts of glycosylation, clarification of this issue and the spectrum of activities exhibited by one molecule requires additional structural data, e.g., substantially full-length sequence analysis of the molecules in question. Protein sequencing offers, of course, a possible means to solve the problem, but it is very difficult work experimentally and often can provide neither completely accurate nor full-length amino acid sequences. Moreover, having the capability of making bulk quantities of a polypeptide exhibiting human GM-CSF activity will greatly facilitate the study of the biology of granulocytes, macrophages, and other cells involved in the immune response; e.g., by minimizing the necessity of relying on ConA-conditioned media for stimulating cell growth. Accurate and complete sequence data on any human CSF will also serve to simplify the search for other immunological factors. Finally, additional information on any lymphokine will help in evaluating the roles of the various growth factors and cells of the immune network and thus provide insight into the entire immune system - with the concomitant therapeutic benefits.

Thus, there exists a significant need for extensive nucleotide sequence data on the DNAs coding for, and amino acid sequences of, proteins exhibiting human GM-CSF activity, as well as a simple and economic method of making substantial and essentially pure quantities of such materials. The present invention fulfills these needs.

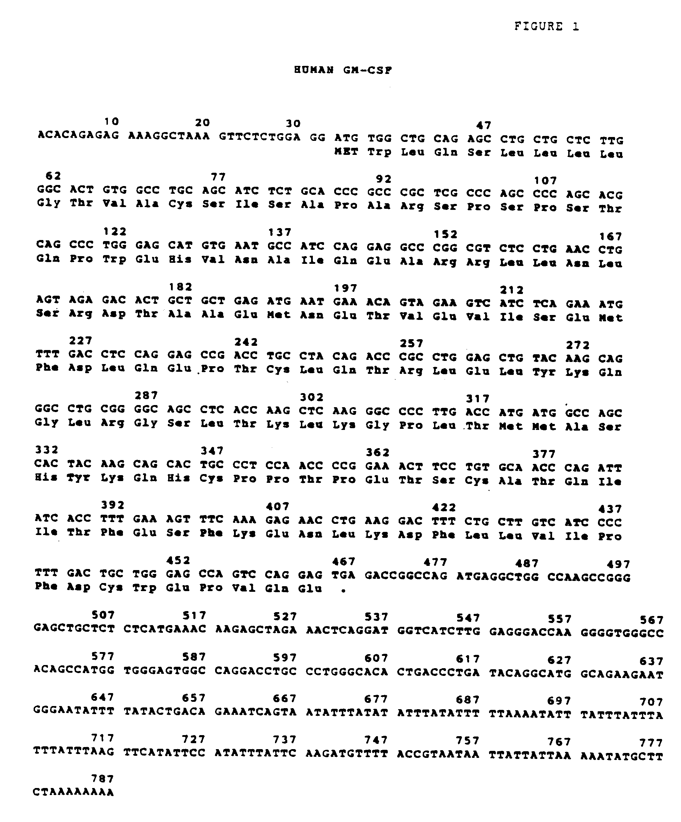

The present invention provides cDNA clones coding for polypeptides exhibiting human GM-CSF activity. A nucleotide sequence for a cDNA and a putative amino acid sequence for an associated polypeptide are shown in Figure 1. The cDNA sequence can be integrated into various vectors, which in turn can direct the synthesis of the corresponding polypeptides in a variety of hosts, including eukaryotic cells, such as mammalian cells in culture.

More specifically, the invention provides a process for producing a polypeptide exhibiting human GM-CSF activity, the process comprising the steps of:

a) providing a vector comprising a nucleotide sequence coding for said polypeptide, wherein the nucleotide sequence is capable of being expressed by a host containing the vector;

b) incorporating the vector into the host; and

c) maintaining the host containing the vector under conditions suitable for expression of the nucleotide sequence into said polypeptide.

Preferably, the cDNA sequences are derived from a nontransformed human T cell's mRNA sequence coding for the polypeptides, and the host is an organism such as a eukaryotic, e.g. mammalian, cell transfected or transformed with the vector. Further, the vector preferably comprises also a second nucleotide sequence capable of controlling expression of the nucleotide sequence coding for the polypeptide. This second sequence coding can include a promoter sequence, one or more intron sequences and a polyadenylation sequence, to permit, respectively, transcription, splicing and polyadenylation of the nucleotide sequence coding for the polypeptide. Particularly, when the host is a mammalian cell, such as a COS-7 monkey (kidney) cell, the vector contains the promoter sequence of the simian virus 40 (SV40) early region promoter and the polyadenylation sequence of the SV40 late region polyadenylation sequence.

The human cDNA sequence of Figure 1 (see below) is capable of hybridizing with other DNA sequences, such as DNA coding for other mammalian growth factors from a cDNA or genomic library. It is noted that the described cDNA sequences seem to contain information for a leader sequence.

The polypeptides of the present invention are capable of enhancing human neutrophilic granulocyte, macrophage, and other cell growth (e.g., eosinophil), particularly in in vitro cultures. Suitable pharmaceutical compositions can be prepared by adding the polypeptides (preparations of which are essentially free of other human growth factors) to therapeutically compatible carriers.

Other features and advantages of the invention will become apparent from the following detailed description, which describes, in conjunction with the accompanying drawings and by way of example, the present invention. In the Drawings:

Figure 1 illustrates the nucleotide sequence and putative corresponding amino acid sequence of a cDNA clone exhibiting human GM-CSF activity;

Figure 2A illustrates pcD-human-GM-CSF, a plasmid carrying a cDNA clone exhibiting human GM-CSF activity;

Figure 2B is a restriction endonuclease cleavage map of the cDNA insert of Figure 2A; and

Figure 3 represents a comparison between putative murine and human GM-CSF amino acid sequences.

In accordance with the present invention, cDNA clones are provided that code for polypeptides exhibiting human GM-CSF activity. After the cDNA sequences have been incorporated into replicable expression vectors, and the vectors transfected into an appropriate host (e.g. a mammalian cell culture), the expressed polypeptide or polypeptides have the ability to allow the expansion of neutrophilic granulocytes, macrophages and other hematopoietic lineages (e.g., eosinophils).

An exemplary, putative amino acid sequence based on the isolated nucleotide sequence is shown in Figure 1. The human GM-CSF cDNA contains a single open-reading frame consisting of 144 codons. Downstream of the putative initiation codon is a region rich in hydrophobic amino acids. It is likely, therefore, that the mature form of secreted human GM-CSF begins with a serine residue, and the preceeding 23 amino acids constitute a leader region, which is subject to removal by proteolytic processing. Thus, in one embodiment human GM-CSF would consist of about 121 amino acids, with a calculated molecular weight of approximately 13,500 daltons (unglycosylated). There appear to be two potential N-glycosylation sites (Asn-X-Ser/Thr) (Neuberger, A. et al., Glycoproteins 5, 450-490, Elsevier Publishing Co., U.S.A. [1972]) at positions 44-46 and 54-56, which could account for the molecular weight discrepancies in the literature.

When transfected into COS-7 monkey cells or other suitable expression systems, cDNA clones of this invention can direct the synthesis of biologically active human GM-CSF. Addition of this expressed cloned gene product to cultures of hematapoietic progenitor cells allows expansion of receptive cells and/or their maintenance in culture. The expressed polypeptides can exhibit the assorted activities associated with a human GM-CSF.

A variety of methods may be used to prepare the cDNAs of the present invention. By way of example, total mRNA is extracted (e.g., as reported by Berger, S. et al., Biochemistry 18: 5143-5149 [1979]) from cells (e.g., a nontransformed human T cell source) producing polypeptides exhibiting human GM-CSF activity. The double-stranded cDNAs from this total mRNA can be constructed by using primer-initiated reverse transcription (Verma, I., Biochim. Biophys. Acta, 473: 1-38 [1977]) to make first the complement of each mRNA sequence, and then by priming for second strand synthesis (Land, H. et al., Nucleic Acids Res., 9: 2251-2266 [1981]). Subsequently, the cDNAs can be cloned by joining them to suitable plasmid or bacteriophage vectors (Rougeon, F. et al., Nucleic Acids Res., 2, 2365-2378 [1975] or Scherer, G. et al., Dev. Biol. 86, 438-447 [1981]) through complementary homopolymeric tails (Efstratiadis, A. et al., Cell, 10, 571-585 [1977]) or cohesive ends created with linker segments containing appropriate restriction sites (Seeburg, P. et al., Nature, 270, 486-494 [1977] or Shine, J. et al., Nature, 270, 494-499 [1977]), and then transforming a suitable host. (See generally Efstratiadis, A., and Villa-Kormaroff, L., "Cloning of double stranded cDNA" in Setlow, J. and Hollaender, A. (eds.) Genetic Engineering, Vol. 1, Plenum Publishing Corp., N.Y., U.S.A. [1982].)

A preferred method of obtaining the full-length cloned cDNAs of this invention is the procedure developed by H. Okayama and P. Berg (Mol. and Cell. Biol., 2: 161-170 [1982]). This method has the advantage of placing the cDNA inserts in a bacterial cloning vector at a position whereby the cDNA can also be directly translated and processed in mammalian cells. Briefly, the first cDNA strand is primed by polydeoxythymidylic acid covalently joined to one end of a linear plasmid vector DNA. The plasmid vector is then cyclized with a linker DNA segment that bridges one end of the plasmid to the 5' end of the cDNA coding sequence. By employing a DNA fragment containing the Simian Virus 40 (SV40) early region promoter and a linker containing a modified SV40 late region intron, the cDNA can be expressed in vitro in COS-7 mouse (kidney) cells without further modification. (See generally Okayama, H. and Berg, P., Mol. and Cell. Biol., 3: 280-289 [1983] and Jolly, D. et al., Proc. Nat. Acad. Sci. U.S.A., 80: 477-481 [1983].)

Once the cDNA library in the Okayama/Berg plasmid vector has been completed, the cDNA clones are collected, and random pools can be checked for the presence of the desired cDNAs by hybrid selection, translation, and assay (e.g. by measuring human GM-CSF activity on cell cultures, the existence of antigenic determinants, or other biological activities). Pools positive by these criteria can then be probed with an appropriate subtracted probe, e.g., cDNA from a B cell line and/or uninduced T cell line. Thereafter, the positive, probed pools are divided into individual clones which are tested by transfection into a suitable host (such as a mammalian cell culture), and the host supernatant assayed for the desired activity. Positive clones are then sequenced.

The desired cDNA clones can also be detected and isolated by hybridization screening with appropriate mRNA samples (Heindell, H. et al., Cell, 15: 43-54 [1978]). Alternatively, the cDNA libraries can be screened by hybrid selection (Harpold, M. et al., Nucleic Acid Res., 5: 2039-2053 [1978] or Parnes, J. et al., Proc. Nat. Acad. Sci. U.S.A., 78: 2253-2257 [1981]) or in Xenopus oocytes (Aurdon, J., Nature, 233: 177-182 [1971]). (See generally Villa-Komaroff, L. et al., Proc. Nat. Acad. Sci. U.S.A., 75: 3727-3731 [1978].)

In further describing the procedures relating to preparing cDNA clones of the invention, the cellular source and other lines will be considered first, followed by general descriptions of the procedures for isolating mRNA coding for a protein exhibiting human GM-CSF activity; the construction of a cDNA library containing the cDNA sequences; isolation of full-length cDNA clones in a plasmid vector and subsequent expression in mammalian cells; subcloning and expression in bacteria and yeast; and purification and formulation. A more detailed description of the entire experimental process will follow thereafter.

T Cell and Other Lines

Any of a large variety of different cells may be used as sources for human GM-CSF activity and in the assay thereof (see, e.g., Burgess, A., Growth Factors and Stem Cells, Academic Press, pgs. 43-124, New York [1984]. A preferred source is a T cell helper line, but human peripheral blood (Verma, D. et al., Brit. J. of Haemat. 57: 505-520 [1984] and Hasketh, P. et al., Blood 63: 1141-1146 [1984]), macrophages or other cells producing human GM-CSF activity (Bodeker, B. et al., Immunobiol. 166: 12-23 [1984]) are acceptable. This includes hybridomas and transformed cell lines (Gasson, J. et al., "Lymphokines and Hematopoiesis", Normal and Neoplastic Hematopoiesis, Alan R. Liss, Inc., New York [1983]).

A preferred source for use in connection with human GM-CSF activity assays is human bone marrow taken from donors not suffering from hematologic deseases. However, human cord blood and spleen are also suitable sources provided they are used within about 12-48 hours after extraction.

To determine CSF activity, the hemopoietic cell source is made into a single cell suspension. The individual cells are then immobilized in a semi-solid (agar) or viscous (methycellulose) medium containing nutrients and usually fetal calf serum. In the presence of an appropriate stimulating factor, individual cells will proliferate and differentiate. Since the initial cells are immobilized, colonies develop as the cells proliferate and mature. These colonies can be scored after 7-14 days. (Burgess, A., Growth Factors and Stem Cells, pgs. 52-55, Academic Press, New York [1984].) (For specific application to the growth of granulocytes and macrophages, see Bradely, T. and Metcalf, D., Aust. J. Exp. Biol. Med. Sci. 44:287-300 [1966], and see generally Metcalf, D., Hemopoietic Colonies, Springer-Verlag, Berlin [1977]). If desired, individual colonies can be extracted, placed on microscope slides, fixed and stained with Wright/Geimsa (Todd-Sanford, Clinical Diagnosis by Laboratory Methods, 15th Edition, Eds. Davidson and Henry [1974]). Morphological analysis of cell types present per single colony can then be determined.

## Isolation of mRNA and Construction of a cDNA Library

Total cellular mRNA can be isolated by a variety of well-known methods (e.g., Przybla, A. et al., J. Biol. Chem. 254: 2154-2158 [1979]), but the preferred method is the guanidinium-thiocyanate extraction procedure of Chirgwin et al. (Biochemistry, 18: 5294-5299 [1979]). If this method is used, approximately 10 $\mu$g of polyA$^+$ mRNA, selected on columns of oligo (dT) cellulose, is obtained from 1-2 x $10^8$ activated human helper T cells.

The cDNA library from the polyA$^+$ mRNA can best be constructed using the pcDVI vector-primer and the pLI linker fragment [available from P-L Biochemicals Inc., Milwaukee, WI] according to procedures which result in greatly enriched full-length copies of mRNA transcripts (e.g. Okayama, H. and Berg, P., Mol. Cell Biol., 2, 161-170 [1982] and Mol. Cell Biol., 3, 280-289 [1983]). The plasmid vector, which contains SV40 early promoter and SV40 RNA processing signals, is designed to promote expression of the cloned cDNA segment in mammalian cells.

Using the Okayama and Berg procedure, the cyclized vector-cDNA preparation is transformed into a competent bacterial cell, such as E. coli MC1061 cells (Casadaban, M. and Cohen, S., J. Mol. Biol., 138: 179-207 [1980]) using calcium chloride (Cohen, S. et al., Proc. Nat. Acad. Sci. U.S.A., 69: 2110-2114 [1972]-). Starting with 5 $\mu$g of polyA$^+$ RNA from ConA-stimulated T cells, about 1.5 x $10^6$ independent transformants can be obtained. Usually, about $10^4$ clones are picked up individually and inoculated into wells of microtiter plates (Flow Laboratories Inc., McLean, Virginia) containing 200 $\mu$l of L-broth, 50 $\mu$g/ml of ampicillin, and 7% DMSO. If desired, sublibraries based on the size of cDNA insert are prepared from total cDNA library as described by Okayama, H. and Berg, P. (Mol. Cell Biol., 3, 280-289 [1983]). Briefly, plasmid DNA is digested with SalI, ClaI, and HindIII separately, and electrophoresed in 1% agarose gel. After staining with ethidium bromide, the gel is sliced into 7 sections corresponding to cDNA insert sizes of 0 to 1, 1 to 2, 2 to 3, 3 to 4, 4 to 5, 5 to 6, and more than 6 kilobases (kb). DNA is extracted from each slice, recycled with T4 DNA ligase, and used to transform MC1061. All nucleotide sequencing can be performed according to the procedure of Maxam, A. and Gilbert, W. (Methods Enzymol., 65: 499-560 [1980]).

## DNA Transfections into Monkey Cells:

Approximately 1x$10^6$ COS-7 monkey kidney cells are seeded onto 60 mm plates the day prior to transfection. Transfections are best performed with 15 $\mu$g of plasmid DNA in 1.5 ml of DME containing 50 mM Tris.HCl, pH 7.4, and 400 $\mu$g/ml DEAE-Dextran (Pharmacia Fine Chemicals, Uppsala, Sweden). This solution is then removed after 4 hr and replaced with 2.0 ml DME + 4% fetal calf serum. The medium is collected after 72 hr and assayed for human GM-CSF IL-2 activity as described above. DNA transfections may be carried out in L-cells and a variety of other cell sources as well (see below).

## Isolation of Related Genes

cDNA clones of the present invention can be used to identify and isolate nucleic acid sequences encoding related genes. Because of the frequent low degree of homology between homologous genes, the stringency of hybridization conditions must be adjusted to allow for cross-hybridization between sequences which may be only 70-80% homologous.

Several different experimental protocols may been used to locate the related genes. For example, the human Ck immunoglobulin light chain gene has been isolated using the corresponding mouse Ck gene as a probe (Heiter, P. et al., Cell 22: 197-207 [1981]) and mouse transplantation antigen genes have been isolated by hybridization to DNA clones encoding their human counterparts (Steinnetz, T. et al., Cell 24: 125-134 [1981]).

For genomic DNA, a preferred method entails plating phage clones from a DNA library containing the homologous genes (Maniatis, T. et al., "Molecular Cloning, A Laboratory Manual", Cold Spring Harbor Laboratory, U.S.A. [1982]) at a density of $2 \times 10^4$ to $5 \times 10^4$ plaques per 150 mm plate on an appropriate host strain such as E. coli LE392. Ten to twenty plates are generally sufficient.

After 10-12 hours' incubation at 37°C, the plates are refrigerated for two hours and then a 132 mm nitrocellulose filter is applied to the agar surface of each plate. The filter is allowed to remain in contact with the plate for at least five minutes, during which time the filters are keyed to the plates by puncturing with an ink-filled 22-gauge needle. The filters are then peeled from the plates and incubated successively for at least two minutes first in 250 ml of 0.1 N NaOH, 0.5 M NaCl; then in 250 ml of 0.5 M Tris.HCl pH 7.5, 1.5 M NaCl. The filters are dried on paper towels and then baked at 80°C for 4-8 hours.

For hybridization, the filters are wetted in 1x SET (0.15 M NaCl, 30 mM Tris.HCl pH 8.0, 1 mM $Na_2$EDTA), then incubated in a solution of 3x SET, 5x Denhardt's (Denhardt, D.T., B.B.R.C. 23: 641-646 [1966]), 10% dextran sulfate, 0.1% sodium dodecyl sulfate (SDS), and 50 $\mu$g/ml each poly (rA), poly (rC), and poly (rG), at 65°C for 2 hrs (1.5-2 ml/filter) with constant agitation. This solution is then discarded, and the filters are hybridized with 0.5 $\mu$g (> $10^8$ cpm) of a nick-translated probe made from GM-CSF cDNA in the same solution (fresh), 1.5-2 ml/filter at 65°C for 1 hour and then at 55°C for 12-20 hours. The filters are then washed successively in 3x SET, 1x Denhardt's; 0.1% SDS; and 1x SET, 0.1% SDS (10-15 ml/filter) at 55°C for one hour with gentle agitation. The filters are dried on paper towels, then autoradiographed for 12-24 hours with appropriate film and an intensifying screen. Hybridizing plaques are picked from the agar plates with sterile pasteur pipets, and each is expelled into 1 ml of 0.1 M NaCl, 0.01 M Tris.HCl pH 7.5, 10 mm MgCl$_2$, 100 $\mu$g/ml gelatin, with 50 $\mu$l of CHCl$_3$ added. After a least 4-8 hours in the cold, the phages from each plaque are rescreened at low density (2000-4000 plaques/150 mm plate) by a procedure identical to that described above.

## Expression in E. coli, in Yeast and in Cell Culture

Prokaryotes, such as E. coli, are very suitable for expression of the polypeptides of the present invention (see, for example, U.S. patent numbers 4,338,397 and 4,411,994), provided glycosylation is not desired.

To obtain high expression levels, promoters should be utilized, such as the $\beta$-lactamase (penicillinase) and lactose promoter systems (Chang et al., Nature, 275: 615 [1978]; Itakura et al., Science, 198: 1056 [1977]; Goeddel et al., Nature 281: 544 [1979] or a tryptophan (trp) promoter system (Goeddel et al., Nucleic Acids Res., 8: 4057 [1980]) in conjunction with Shine-Delgarno sequences.

Those skilled in the art will realize that not only prokaryotes but also eukaryotic microbes, such as yeast, may also be used in protein production. Saccharomyces cerevisiae is a preferred eukaryotic microorganism. Suitable promoting sequences in yeast vectors include the promoters for 3-phosphoglycerate kinase (Hitzeman et al., J. Biol. Chem., 255: 12073-12080 [1980]) or other glycolytic enzymes (Hess et al., Adv. Enzyme Reg., 7: 149-167 [1969]; Holland et al., Biochemistry, 17: 4900-4907 [1978]). Other promoters that have the additional advantage of transcription controlled by growth conditions may be used. Basically any plasmid vector containing a yeast-compatible promoter, an origin of replication and termination sequences is suitable.

A preferred method of making human GM-CSF employing the cDNA's of the present invention utilizes the yeast mating pheromone $\alpha$-factor secretory pathways (Julius, D. et al., Cell 32: 839-852 [1983]). S. cerevisiae secretes mating-type specific oligopeptide pheromones. MAT$\alpha$ cells secrete $\alpha$-factor, which induces the growth arrest of MAT$\alpha$ cells at G1 phase of the cell cycle (Thorner, J., The Molecular Biology of the Yeast Saccharomyces, Cold Spring Harbor Laboratory, New York [1981]; see particularly pages 143-180). The $\alpha$-factor is initially synthesized as a larger precursor molecule consisting of an NH$_2$-terminal signal sequence of about 20 amino acids, followed by an additional 60 amino acids leader sequence and ending with four identical tandem repeats of the mature $\alpha$-factor sequence. The repeats are separated from each other by six or eight amino acids spacers (Lys-Arg-Glu-Ala-Glu-Ala and Lys-Arg-Glu-Ala-Glu-[or Asp-]-Ala-Glu-Ala). This prepro-$\alpha$-factor is cleaved at several specific sites. The first processing is the cleavage of the COOH-terminal side of the Lys-Arg pair of the spacer sequence catalysed by the KEX2 product (Julius et al., Cell 37: 1075-1089 [1984]). A carboxypeptidase-B like enzyme cleaves at the NH$_2$-terminal side of the Lys-Arg pair. The final step is the removal of Glu-Ala or Asp-Ala pairs by diaminopeptidase, which is encoded by the STE13. Brake, J. et al. (Proc. Nat. Acad. Sci. U.S.A. 81: 4642-4646 [1984]) have shown that the fusion of the sequence encoding mature human proteins to the first processing site allowed secretion of such proteins.

A general yeast expression vector, designated pMF-alpha-8, containing the alpha factor promoter and downstream leader sequence in conjunction with other elements, has been deposited with the ATCC (accession number 40140). It can be constructed as follows:

A 1.7 kb EcoRI fragment carrying the MFα1 gene (Kurjan, J. and Hershowitz, I., Cell. 30: 933-943 [1982]) is cloned into the EcoRI restriction site of M13mp8 (Viera, J. and Messing, J., Gene 19: 259-268 [1982]). In order to introduce a HindIII site after the lysine codon of the first spacer region, the synthetic oligonucleotide TCTTTTATCCAAAGATACCC is hybridized to the single strand M13-MFα1 DNA and the oligonucleotide primer extended by DNA polymerase I Klenow fragment. After SI nuclease treatment, the DNA is cleaved with EcoRI and the fragment carrying the MFα1 promoter and leader sequence cloned into the EcoRI and filled-in HindIII restriction sites of pUC8 (Viera, J. and Messing, J. above). One plasmid with the desired structure can be isolated (designated pMFα4ΔI). The pMFα4ΔI is cleaved with HindIII and partially filled in with DNA polymerase I Klenow fragment in the presence of dATP and dGTP. The DNA is treated with mung bean nuclease, and the oligonucleotide linker GCCTCGAGGC attached. The resultant plasmid (designated pMFα5) will have a StuI cleavage site immediately after the arginine codon, followed by the XhoI restriction site. An S. cerevisiae-E. coli shuttle vector (pTRP584) can be constructed as follows: the PstI-XbaI fragment carrying 2 μm plasmid replication origin (Broach, J. above) is cloned into the ClaI restruction site of pTRP56 (Miyajima et al., Mol. Cell. Biol. 4: 407-414 [1984]) and the StuI restriction site within the TRPI-ARSI fragment converted into a PvuII restriction site by PvuII linker insertion. The KpnI restriction site in the original pTRP56 is converted to XhoI by the XhoI linker insertion. The general secretion vector pMFα8 is then obtained by insertion of the BglII-XhoI fragment of pMFα5 into the BamHI-XhoI restriction sites of pTRP584.

Those skilled in the art will realize that cDNA clones encoding for human GM-CSF may then be readily inserted into the pMFα8 vector and subsequently transformed in yeast for human GM-CSF production. By way of example, a 1.0 kb Bam HI fragment carrying the entire human GM-CSF cDNA is cloned into the Bam HI restriction site of M13mp8 (Viera, J. and Messing, J., Gene 19:259-268 [1982]). In order to make a double stranded fragment carrying the mature protein coding sequence, an oligonucleotide primer AGCCCCAGCACGCAGCCCTGGGAGCAT is constructed. This primer is then hybridized to a single stranded M13mp8 carrying the human GM-CSF cDNA, and is extended by DNA polymerase I Klenow fragment. The double stranded DNA is cleaved with Bam HI and then the single strand region is removed by mung bean nuclease. The double stranded fragment carrying human GM-CSF mature protein coding sequence is then isolated and cloned into the StuI restriction site of the general secretion vector pMFχ8. This plasmid DNA (carrying the TRPI gene) can be introduced into yeast cells by the lithium acetate method (Ito, H. et al., J. Bacteriol. 153: 163-168 [1983]) and transformants selected in synthetic medium lacking tryptophan. Transformants are then grown in a common medium supplemented with 0.5% casamino acids. To harvest the yeast cells, they are first resuspended in phosphate-buffered-saline (PBS) containing 1 mM PMSF and then disintegrated by vigorous shaking with acid washed glass beads. Clear supernatant is obtained by centrifugation at 10,000 rpm for 15 min.

In addition to microorganisms, cell cultures derived from multicellular organisms (especially mammalian cells) may also be used as hosts. Examples of such useful host cell lines are HeLa cells, Chinese hamster ovary cell lines, and baby hamster kidney cell lines. Expression vectors for such cells ordinarily include, as necessary, an origin of replication, a promoter located in front of the gene to be expressed, along with any required ribosome binding sites, RNA splice sites, polyadenylation sites, and transcriptional terminator sequences. When used in mammalian cells, the expression vector often has control functions provided by viral material. For example, commonly used promoters are derived from polyoma, Adenovirus 2, and most frequently SV-40. (See, e.g., U.S. patent No. 4,399,216 and Gheysen, D. and Fiers, W., J. of Mol. and Appl. Genetics 1: 385-394 [1982].)

Purification and Formulations

The human GM-CSF polypeptides expressed in E. coli, in yeast or in other cells can be purified according to standard procedures of the art, including ammonium sulfate precipitation, fractionation column chromatography (e.g., ion exchange, gel filtration, electrophoresis, affinity chromatography, etc.) and ultimately crystallization (see generally "Enzyme purification and Related Techniques", Methods in Enzymology, 22: 233-577 [1977] and Scopes, R., Protein Purification: Principles and Practice, Springer-Verlag, New York [1982]). Once purified, partially or to homogeneity, the polypeptides of the invention may be used for research purposes, e.g., as a supplement to cell growth media (e.g., minimum essential medium Eagle, Iscove's modified Dulbecco Medium or RPMI 1640; available from Sigma Chemical Company, St. Louis, MO and GIBCO Division, Chagrin Falls, OH) and as an antigenic substance for eliciting specific immunoglobulins useful in immunoassays, immuno-fluorescent stainings, etc. (See generally Immunological Methods, Vols. I & II, Eds. Lefkovits, I. and Pernis, B., Academic Press, New York, N.Y. [1979 & 1981]; and Handbook of Experimental Immunology, ed. Weir, D., Blackwell Scientific Publications, St. Louis, MO [1978].)

The polypeptides of the present invention may also be used in pharmaceutical compositions, e.g. as an enhancement of natural defense against various neoplastic and infectious disease states or adjunct in chemotherapy to overcome myelosuppression (see, Gasson, J. et al., "Lymphokines and Hematopoiesis" in Normal and Neoplastic Hematopoisesis, pgs. 129-139, Alan R. Liss, Inc. New York [1983]).

For preparing pharmaceutical compositions containing the polypeptides described by this invention, these polypeptides are compounded by admixture with preferably inert, pharmaceutically acceptable carriers. Suitable carriers and processes for their preparation are well known in the art (see e.g. Remington's Pharmaceutical Sciences and U.S. Pharmacopeia: National Formulary, Mack Publishing Company, Easton, PA [1980]). The preferred course of administration is parenteral and can include use of mechanical delivery systems.

Preferably, the pharmaceutical composition is in unit dosage form. In such form, the preparation is subdivided into unit doses containing appropriate quantities of the active component. The quantity of active compound in a unit dose of preparation may be varied or adjusted from 1 $\mu$g to 100 mg, according to the particular application and the potency of the active ingredient. The composition can, if desired, also contain other therapeutic agents.

The dosages may be varied depending upon the requirement of the patient, the severity of the condition being treated and the particular compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day.

The following experimental information and data are offered by way of example and not by way of limitation.

EXPERIMENTAL

A. Cloned Human Helper T Cells
1) A clone of human T Cells designated T-7 was isolated according to the method described in Chapters 36 and 37 of Isolation, Characterization, and Utilization of T Lymphocyte Clones, Eds. Fathman, C. and Fitch, F., Academic Press, New York (1982). The cell line was continuously maintained at about 0.5 x $10^5$ cells/ml in Dulbeco's Modified Eagle (DME) medium with 10% heat-inactivated fetal calf serum, 5 x $10^{-5}$ M 2-ME, 2 mM glutamine, non-essential amino acids, and essential vitamins conditioned with 30% supernatants from phytohemagglutinin (PHA) stimulated human peripheral leukocytes.
2) PHA-activation of T-7 cells: The cells were cultured at 5 x $10^5$/ml in DME with 4% heat-inactivated fetal calf serum, 5 x $10^{-5}$ M 2-ME, 2mM glutamine, non-essential amino acids, essential vitamins and 4 $\mu$g/ml ConA. After 4-6 hrs.' incubation at 37°C in 10% $CO_2$, the cell suspension was centrifuged at 1500 rpm for 10 minutes. The cell pellets were collected and frozen immediately at -70°C. The supernatants were filtered (Nalgene-0.22 microns) and stored at -80°C as a source of growth factors. Aliquots of the supernatant were assayed for CSF activity (see below) to verify the induction of the line by the PHA treatment.
B. Bone Marrow and Cord Blood Assays
Bone marrow cells collected from patients with nonhematologic disease were layered over Ficoll (type 400, Sigma Chemical Co., St. Louis, MO), centrifuged (2,000 rpm, 20 min), and the cells at the interface removed. These cells were washed twice in Iscove's Modified Dulbeccos' Medium containing 10% fetal calf serum (FCS), resuspended in the same medium and the adherent cells removed by adherence to plastic Petri dishes. The nonadherent cells were added at $10^5$ cells/ml to Iscove's Medium containing 20% FCS, 50 $\mu$M 2-mercaptoethanol, 0.9% methylcellulose and varied concentrations of either supernatants known to contain colony stimulating activity or test supernatants. One ml aliquots were plated in 35 mm petri dishes and cultured at 37°C in a fully humidified atmosphere of 6% $CO_2$ in air. Three days after the initiation of the culture, 1 unit of erythropoietin (Eaves, A. British Columbia Cancer Research Center, Vancouver, B.C.) was added to each plate. Granulocyte-macrophage colonies and erythroid bursts were scored at 10-14 days using an inverted microscope.

Cord blood cells collected in heparin were spun at 2,000 rpm for 6 min. The white blood cells at the interface between the plasma and red blood cell peak was transferred to a tube containing 0.17 N ammonium chloride and 6% FCS. After 5 min on ice, the suspension was underlaid with 4 ml FCS and centrifuged for 6 mins at 2,000 rpm. The cell pellet was washed with Dulbecco's phosphatge buffered saline and put through the Ficoll and plastic adherence steps as described above for bone marrow cells. The low density nonadherent cells were collected and placed at $10^5$ cells/culture in the semi-solid agar system as described above.

At the end of the assays, the cellular composition was determined after applying the individual colonies to glass slides and staining with Wright-Giemsa. Eosinophils were determined by staining with Luxol Fast Blue (Johnson, G. and Metcalf, D., Exp. Hematol. 8: 549-561 [1980]).

C. Isolation of mRNA from Human Cells.

1) Total cellular RNA was isolated from cells using the guanidine isothiocyanate procedure of Chirgwin, J. et al., (Biochemistry, 18: 5294-5299 [1979]).

Frozen cell pellets from uninduced or ConA-induced human helper cells (4 hrs after stimulation) were suspended in guanidine isothiocyanate lysis solution. Twenty ml of lysis solution was used for $1.5 \times 10^8$ cells. Pellets were resuspended by pipetting, then DNA was sheared by 4 passes through a syringe using a 16 gauge needle. The lysate was layered on top of 20 ml of 5.7 M CsCl, 10 mM EDTA in 40 ml polyallomer centrifuge tube. This solution was centrifuged at 25,000 rpm in a Beckman SW28 rotor (Beckman Instruments, Inc., Palo Alto, CA) for 40 hrs at 15°C. The guanidine isothiocyanate phase containing DNA was pipetted off from the top, down to the interface. The walls of the tube and interface were washed with 2-3 ml of guanidine isothiocyanate lysis solution. The tube was cut below the interface with scissors, and the CsCl solution was decanted. RNA pellets were washed twice with cold 70% ethanol. Pellets were then resuspended in 500 $\mu$l of 10 mM Tris.HCl pH 7.4, 1 mM EDTA, 0.05% SDS. 50 $\mu$l of 3M sodium acetate was added and RNA was precipitated with 1 ml ethanol. About 0.3 mg total RNA was collected by centrifuging and the pellets washed once with cold ethanol.

2) PolyA$^+$ mRNA isolation:

Washed and dried total RNA pellet was resuspended in 900 $\mu$l of oligo (dT) elution buffer (10 mM Tris.HCl, pH 7.4, 1 mM EDTA, 0.5% SDS). RNA was heated for 3 min. at 68°C and then chilled on ice. 100 $\mu$l of 5 M NaCl was added. The RNA sample was loaded onto a 1.0 ml oligo (dT) cellulose column (Type 3, Collaborative Research, Waltham, MA) equilibrated with binding buffer (10 mM Tris.HCl pH 7.4, 1 mM EDTA, 0.5 M NaCl, 0.5% SDS.). Flow-through from the column was passed over the column twice more. The column was then washed with 20 ml binding buffer. PolyA$^+$ mRNA was collected by washing with elution buffer. RNA usually eluted in the first 2 ml of elution buffer. RNA was precipitated with 0.1 volume 3 M sodium acetate (pH 6) and two volumes of ethanol. The RNA pellet was collected by centrifugation, washed twice with cold ethanol, and dried. The pellet was then resuspended in water. Aliquots were diluted, and absorbance at 260 nm was determined.

D. cDNA Library Construction:

1) Preparation of vector primer and oligo dG-tailed linker DNAs:

The procedure of Okayama & Berg (Mol. & Cell. Biol. 2: 161-170 [1982]) was used with only minor modifications and adapted to the pcDVI and pLl plasmids described by Okayama & Berg (Mol. & Cell. Biol. 3: 380-389 [1983]).

An 80 $\mu$g sample of pcDVI DNA was digested at 30°C with 20 U of KpnI endonuclease in a reaction mixture of 450 $\mu$l containing 6 mM Tris.HCl (pH 7.5), 6 mM MgCl$_2$, 6 mM NaCl, 6 mM 2-ME, and 0.1 mg of bovine serum albumin (BSA) per ml. After 16 hr the digestion was terminated with 40 $\mu$l of 0.25 M EDTA (pH 8.0) and 20 $\mu$l of 10% sodium dodecyl sulfate (SDS); the DNA was recovered after extraction with water-saturated 1:1 phenol-CHCl$_3$ (hereafter referred to as phenol-CHCl$_3$) and ethanol precipitation. Homopolymer tails averaging 60, but not more than 80, deoxythymidylate (dT) residues per end were added to the KpnI endonuclease-generated termini with calf thymus terminal transferase as follows: The reaction mixture (38 $\mu$l) contained sodium cacodylate-30 mM Tris.HCl pH 6.8 as buffer, with 1 mM CoCl$_2$, 0.1 mM dithiothreitol, 0.25 mM dTTP, the KpnI endonuclease-digested DNA, and 68 U of the terminal deoxynucleotidyl transferase (P-L Biochemicals, Inc., Milwaukee, WI). After 30 min. at 37°C the reaction was stopped with 20 $\mu$l of 0.25 M EDTA (pH 8.0) and 10 $\mu$l of 10% SDS, and the DNA was recovered after several extractions with phenol-CHCl$_3$ by ethanol precipitation. The DNA was then digested with 15 U of EcoRI endonuclease in 50 $\mu$l containing 10 mM Tris.HCl pH 7.4, 10 mM MgCl$_2$, 1 mM dithiothreitol, and 0.1 mg of BSA per ml for 5 hr at 37°C. The large fragment, containing the SV40 polyadenylation site and the pBR322 origin of replication and ampicillin-resistance gene, was purified by agarose (1%) gel electrophoresis and recovered from the gel by a modification of the glass powder method (Vogelstein, B. & Gillespie, D., Proc. Nat. Acad. Sci. 76: 615-619 [1979]). The dT-tailed DNA was further purified by absorption and elution from an oligo (dA)-cellulose column as follows: The DNA was dissolved in 1 ml of 10 mM Tris.HCl pH 7.3 buffer containing 1 mM EDTA and 1 M NaCl, cooled at 0° C, and applied to an oligo (dA)-cellulose column (0.6 by 2.5 cm) equilibrated with the same buffer at 0° C. The column was washed with the same buffer at 0° C and eluted with water at room temperature. The eluted DNA was precipitated with ethanol and dissolved in 10 mM Tris.HCl pH 7.3 with 1 mM EDTA.

The oligo (dG) tailed linker DNA was prepared by digesting 75 $\mu$g of pLl DNA with 20 U of Pstl endonuclease in 450 $\mu$l containing 6 mM Tris.HCl pH 7.4, 6 mM MgCl$_2$, 6 mM 2-ME, 50 mM NaCl, and 0.01 mg of BSA per ml. After 16 hr at 30° C the reaction mixture was extracted with phenol-CHCl$_3$ and the DNA was precipitated with alcohol. Tails of 10 to 15 deoxyguanylate (dG) residues were then added per end with 46 U of terminal deoxynucleotidyl transferase in the same reaction mixture (38 $\mu$l) as described above, except that 0.1 mM dGTP replaced dTTP. After 20 min. at 37° C the mixture was extracted with phenol-CHCl$_3$, and after the DNA was precipitated with ethanol it was digested with 35 U of HindIII endonuclease in 50 $\mu$l containing 20 mM Tris.HCl pH 7.4, 7 mM MgCl$_2$, 60 mM NaCl, and 0.1 mg of BSA at 37° C for 4 hr. The small oligo (dG)-tailed linker DNA was purified by agarose gel (1.8%) electrophoresis and recovered as described above.

2) cDNA Library Preparation:

Step 1: cDNA synthesis. The reaction mixture (10 $\mu$l) contained 50 mM Tris.HCl pH 8.3, 8 mM MgCl$_2$, 30 mM KCl, 0.3 mM dithiothreitol, 2 mM each dATP, dTTP, dGTP, and dCTP, 20 $\mu$Ci $^{32}$P-dCTP (3000 Ci/mmole), 3 $\mu$g polyA$^+$ RNA from Con-A induced T-cells, 60 units RNasin (Biotec, Inc., Madison, WI), and 2 $\mu$g of the vector-primer DNA (15 pmol of primer end), and 45 U of reverse transcriptase. The reaction was incubated 60 min at 42° C and then stopped by the addition of 1 $\mu$l of 0.25 M ETDA (pH 8.0) and 0.5 $\mu$l of 10% SDS; 40 $\mu$l of phenol-CHCl$_3$ was added, and the solution was blended vigorously in a Vortex mixer and then centrifuged. After adding 40 $\mu$l of 4 M ammonium acetate and 160 $\mu$l of ethanol to the aqueous phase, the solution was chilled with dry ice for 15 min., warmed to room temperature with gentle shaking to dissolve unreacted deoxynucleoside triphosphates that had precipitated during chilling, and centrifuged for 10 min. in an Eppendorf microfuge. The pellet was dissolved in 10 $\mu$l of 10mM Tris.HCl pH 7.3 and 1 mM EDTA, mixed with 10 $\mu$l of 4 M ammonium acetate, and reprecipitated with 40 $\mu$l of ethanol, a procedure which removes more than 99% of unreacted deoxynucleotide triphosphates. The pellet was rinsed with ethanol.

Step 2: Oligodeoxycytidylate [oligo (dC)] addition. The pellet containing the plasmid-cDNA:mRNA was dissolved in 20 $\mu$l of 140 mM sodium cacodylate-30 mM Tris.HCl pH 6.8 buffer containing 1 mM CoCl$_2$, 0.1 mM dithiothreitol, 0.2 $\mu$g of poly(A), 70 $\mu$M dCTP, 5 $\mu$Ci $^{32}$P-dCTP, 3000 Ci/mmole, and 60 U of terminal deoxynucleotidyl transferase. The reaction was carried out at 37° C for 5 min. to permit the addition of 10 to 15 residues of dCMP per end and then terminated with 2 $\mu$l of 0.25 M EDTA (pH 8.0) and 1 $\mu$l of 10% SDS. After extraction with 20 $\mu$l of phenol-CHCl$_3$, the aqueous phase was mixed with 20 $\mu$l of 4 M ammonium acetate, the DNA was precipitated and reprecipitated with 80 $\mu$l of ethanol, and the final pellet was rinsed with ethanol.

Step 3: HindIII endonuclease digestion. The pellet was dissolved in 30 $\mu$l of buffer containing 20 mM Tris.HCl pH 7.4, 7 mM MgCl$_2$, 60 mM NaCl, and 0.1 mg of BSA per ml and then digested with 10 U of HindIII endonuclease for 2 hr at 37° C. The reaction was terminated with 3 $\mu$l of 0.25 M EDTA (pH 8.0) and 1.5 $\mu$l of 10% SDS and, after extraction with phenol-CHCl$_3$ followed by the addition of 30 $\mu$l of 4 M ammonium acetate, the DNA was precipitated with 120 $\mu$l of ethanol. The pellet was rinsed with ethanol and then dissolved in 10 $\mu$l of 10 mM Tris.HCl (pH 7.3) and 1 mM EDTA, and 3 $\mu$l of ethanol was added to prevent freezing during storage at -20° C.

Step 4: Cyclization mediated by the oligo (dG)-tailed linker DNA. A 9 $\mu$l sample of the HindIII endonuclease-digested oligo (dC)-tailed cDNA:mRNA plasmid (about 90% of the sample) was incubated in a mixture (90 $\mu$l) containing 10 mM Tris.HCl pH 7.5, 1 mM EDTA, 0.1 M NaCl, and 1.8 pmol of the oligo (dG)-tailed linker DNA at 65° C for 5 min, shifted to 42° C for 60 min, and then cooled to 0° C. The mixture (90 $\mu$l) was adjusted to a volume of 900 $\mu$l containing 20 mM Tris.HCl pH 7.5, 4 mM MgCl$_2$, 10 mM (NH$_4$)$_2$SO$_4$, 0.1 M KCl, 50 $\mu$g of BSA per ml, and 0.1 mM $\beta$-NAD; 6$\mu$g of E. coli DNA ligase were added and the solution was then incubated overnight at 12° C.

Step 5: Replacement of RNA strand by DNA. To replace the RNA strand of the insert, the ligation mixture was adjusted to contain 40 $\mu$M of each of the four deoxynucleoside triphosphates, 0.15 mM b-NAD, 4 $\mu$g of additional E. coli DNA ligase, 16 U of E. coli DNA polymerase I (Poll,) and 9 U of E. coli RNase H. This mixture (960 $\mu$l) was incubated successively at 12° C and room temperature for 1 hr each to promote optimal repair synthesis and nick translation by PolI.

Step 6: Transformation of E. coli. Transformation was carried out using minor modifications of the procedure described by Cohen et al. (Proc. Nat. Acad. Sci. U.S.A., 69: 2110-2114 [1972]). E. coli K-12 strain MC1061 (Casadaban, M. and Cohen, S., J. Mol. Biol. 138: 179-207 [1980]) was grown to 0.5 absorbancy unit at 600 nm at 37°C in 20 ml of L-broth. The cells were collected by centrifugation, suspended in 10 ml of 10 mM Tris.HCl pH 7.3 containing 50 mM $CaCl_2$, and centrifuged at 0°C for 5 min. The cells were resuspended in 2 ml of the above buffer and incubated again at 0°C for 5 min.; then, 0.2 ml of the cell suspensions was mixed with 0.1 ml of the DNA solution (step 5) and incubated at 0°C for 15 min. Next the cells were kept at 37°C for 2 min. and thereafter at room temperature for 10 min.; then 0.5 ml of L-broth was added, and the culture was incubated at 37°C for 30 min., mixed with 2.5 ml of L-broth soft agar at 42°C, and spread over L-broth agar containing 50 $\mu$g of ampicillin per ml. After incubation at 37°C for 12 to 24 hr, individual colonies were picked with sterile tooth-picks. In all, approximately $5 \times 10^4$ independent cDNA clones were generated.

E. Screening of the Human T-cell cDNA Library by DNA Transfections:

A collection of $10^4$ independent clones were picked at random from the T-cell cDNA library and propagated individually in wells of microtiter dishes containing 200 $\mu$l L broth with ampicillin at 50 $\mu$g/ml and dimethyl sulfoxide at 7%. Pools containing 48 cDNA clones were prepared from the microtiter cultures. 40 such pools were grown up in 1 liter cultures of L-broth containing 100 $\mu$g/ml ampicillin. Plasmid DNA was isolated from each culture and purified by twice banding through CsCl gradients. The DNA representing each pool was transfected into COS-7 monkey cells as follows.

One day prior to transfection, approximately $10^6$ COS-7 monkey cells were seeded onto individual 60 mm plates in DME containing 10% fetal calf serum and 2 M glutamine. To perform the transfection, the medium was aspirated from each plate and replaced with 1.5 ml of DME containing 50 mM Tris.HCl pH 7.4, 400 $\mu$g/ml DEAE-Dextran and 15 $\mu$g of the plasmid DNAs to be tested. The plates were incubated for four hours at 37°C, then the DNA-containing medium was removed, and the plates were washed twice with 2 ml of serum-free DME. DME containing 150 $\mu$M chloroquine was added back to the plates which were then incubated for an additional 3 hrs at 37°C. The plates were washed once with DME and then DME containing 4% fetal calf serum, 2 mM glutamine, penicillin and streptomycin was added. The cells were then incubated for 72 hrs at 37°C. The growth medium was collected and assayed for human GM-CSF activity as described above.

Four pools (groups 1A, 3B, 7A, and 14A) yielded human GM-CSF activity (see Table I below). Each group was then subdivided into 6 pools, each containing 8 of the original pooled clones. One subpool from each initial pool was positive in the transfection assay, with the exception of 7A which yielded two positive subpools. Each of the plasmids in four of the five subpools was transfected individually into COS-7 cells. Four single clones, designated 3-8a, 7-1a, 7-4d, and 14-1e were active in producing GM-CSF activity. Restriction endonuclease analysis showed that all of these clones share essentially the same structure.

Table I presents the number of hemopoietic colonies stimulated by each transfection sample in duplicate cord blood assays. A cluster represents 20 to 50 cells, a small colony was 51 to 150 cells, and a colony was more than 150 cells.

13

TABLE I

Assay of Colony Stimulating Activity
from Plasmid DNA Pools Transfections

First
Screening        40 pools of 48 clones (1-20, A + B)

                 Mock-infected COS-7:  7 + 12 clusters

                 Pool  1A:  29 + 25 clusters

                 Pool  3B:  38 + 20 clusters

                 Pool  7A:  22 + 19 clusters

                 Pool 14A:  26 + 32 clusters

                 All other pools:  fewer than 20 clusters

Second
Screening     Sub-pools of 8 clones

```
Mock-infected COS-7:  9 + 15 clusters

Sub-pool  1-5:   56 + 54 clusters

Sub-pool  3-8:   98 + 52 small colonies

Sub-pool  7-1:   29 + 41 small colonies

Sub-pool  7-4:  100 + 93 small colonies

Sub-pool 14-1:   40 + 73 small colonies

All other sub-pools:  fewer than 20
clusters
```

---

```
Third
Screening    Individual clones

             Clone  3 - 8a:  120 + 127 colonies

             Clone  7 - 1a:  198 + 164 colonies

             Clone  7 - 4a:  176 + 160 colonies

             Clone 14 - 1e:   62 +  67 colonies
             All other clones:  fewer than 20 clusters
```

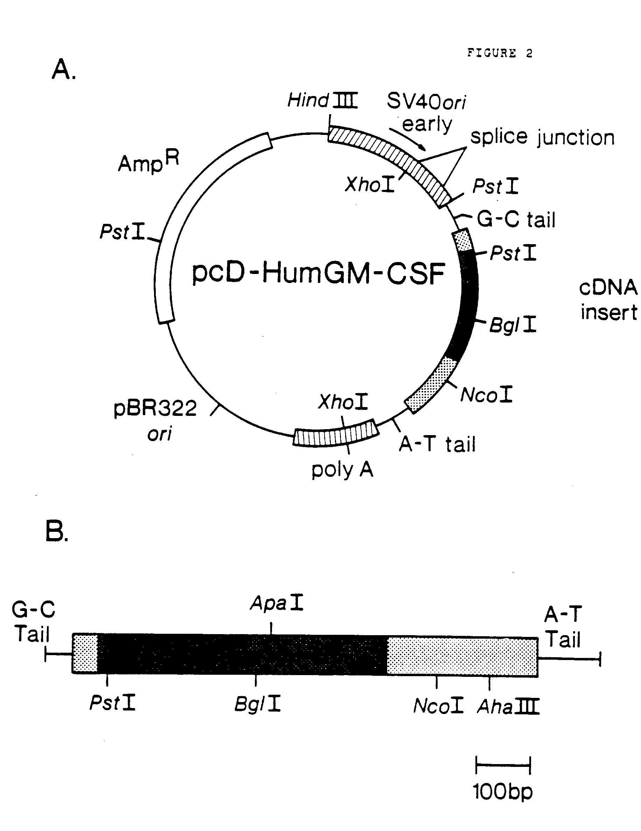

A plasmid (pcD-human-GM-CSF) carrying a substantially full-length cDNA insert is shown in Figure 2, and an E. coli bacterium (MC1061) carrying the plasmid has been deposited with the ATCC (accession number 39923). In Figure 2, transcription of the 776 bp cDNA insert contained in the pcD expression vector from the SV40 early promoter is indicated by the arrow. The locations of the splice donor and acceptor sites are shown. A polyadenylation signal derived from SV40 is located at the 3'-end of the cDNA insert. The GM-CSF coding region in the cDNA insert is heavily shaded, while the non-coding regions are lightly shaded. The remainder of the vector sequences are derived from pBR322, including the $\beta$-lactamase gene (Amp$^R$) and the origin of replication.

Utilizing both the M13 dideoxy chain termination method (Sanger, F., et al., Proc. Natl. Acad. Sci. U.S.A. 74: 5463-5467 [1977]) and modified Maxam/Gilbert technique (Rubin, C. and Schmid, C., Nucleic Acid Res. 8: 4613-4619 [1981]) the 3-8a sequence was determined. The cDNA insert contains a single open reading frame. The first ATG is found 33-35 nucleotides from the 5' end, and is followed by 144 codons before the termination triplet (TGA) at nucleotide positions 465-467.

Table II shows a percentage breakdown of the cellular composition of about 60 human bone marrow and cord blood colonies grown under the influence of the individual clones 3-8a, 7-1a, 7-4d, and 14-1e. The existence of mixed colonies of eosinophils and the other cell types could be due to colonies growing together.

TABLE II

| Cellular Composition of Human Bone Marrow Colonies | | | | | |
|---|---|---|---|---|---|
| Neu | Mθ | Eos | Neu/Mθ | Mθ/Eos | Neu/Mθ/Eos |
| 15% | 30% | 7% | 37% | 2% | 9% |

A full length cDNA (including a signal sequence) encoding murine GM-CSF activity has also been isolated, and an E. coli bacterium (MC1061) carrying a pcD plasmid with the murine cDNA has been deposited with the ATCC (accession number 39924). A $^{32}$P-labeled probe of the PST I/Ala III fragment from the mouse cDNA hybridized under low stringency conditions (42°C) with one of the human GM-CSF cDNA's of the present invention (see Maniatis, T. et al., Molecular Cloning, A Laboratory Manual, Cold Spring Harbor Laboratory [1982]).

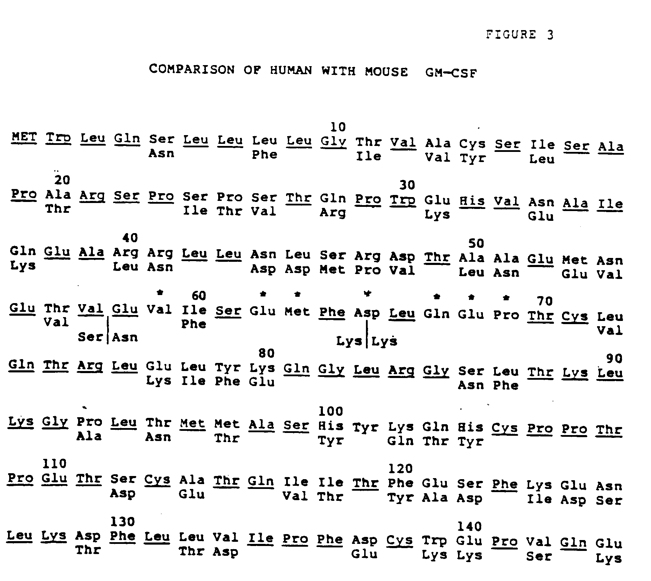

Figure 3 is a comparison between the putative mouse and human GM-CSF protein sequences. Identical residues (after alignment) are underlined, while residues reduced to be present in the human protein but not in mouse protein are indicated by an asterisk, and residues present only in the mouse (i.e., Ser + Asn at positions 57-58 and Lys-Lys at position 65) are indicated by a short vertical line. The homologies between the mouse and human GM-CSF cDNAs are surprising. Overall, there is about 70% homology between the two CM-CSF sequences (Brutlag, D. et al., Nucleic Acids Res. 10: 279-294 [1981]). However, the human GM-CSF of the present invention does not significantly stimulate the growth of mouse hemopoietic stem cells in vitro.

The clone library in the pcD expression vector enabled the identification of complete cDNA clones by direct expression in mammalian cells. Specifically, complete human GM-CSF cDNA clones were directly identified by transfecting COS-7 cells with randomly picked cDNA clones, and measuring the GM-CSF activity secreted into the cell supernatant. These results confirm that the identification of full-length cDNA clones of lymphokines or hormones may be achieved solely on the basis of detection of a functional polypeptide in eukaryotic cells.

From the foregoing, it will be appreciated that the cDNA clones of the present invention provide accurate and complete sequence data on a human GM-CSF. The invention also provides to those skilled in the art means for producing significant quantities of the factor (essentially free from other hematopoietic factors) for the improved in vitro maintenance of neutrophilic granulocytes, and macrophages, as well as other hematopoietic cells (e.g. eosinophils). Further, the information gleaned from the cDNA clones increases understanding of the mammalian immune response, enhancing experimental research capabilities.

**Claims**

**Claims for the following Contracting States : BE, CH, DE, FR, GB, IT, LI, LU, NL, SE**

1.  A recombinant polypeptide exhibiting colony stimulating factor activity on human neutrophilic granulocytes, macrophages, and eosinophils and having the structure

```
                                        Met Trp Leu Gln Ser Leu Leu Leu Leu
Gly Thr Val Ala Cys Ser Ile Ser Ala Pro Ala Arg Ser Pro Ser Pro Ser Thr
Gln Pro Trp Glu His Val Asn Ala Ile Gln Glu Ala Arg Arg Leu Leu Asn Leu
Ser Arg Asp Thr Ala Ala Glu Met Asn Glu Thr Val Glu Val Ile Ser Glu Met
Phe Asp Leu Gln Glu Pro Thr Cys Leu Gln Thr Arg Leu Glu Leu Tyr Lys Gln
Gly Leu Arg Gly Ser Leu Thr Lys Leu Lys Gly Pro Leu Thr Met Met Ala Ser
His Tyr Lys Gln His Cys Pro Pro Thr Pro Glu Thr Ser Cys Ala Thr Gln Ile
Ile Thr Phe Glu Ser Phe Lys Glu Asn Leu Lys Asp Phe Leu Leu Val Ile Pro
Phe Asp Cys Trp Glu Pro Val Gln Glu
```

16

EP 0 202 300 B1

2. A polypeptide according to Claim 1 without a signal sequence.

3. A polypeptide according to Claim 1 or Claim 2 being glycosylated.

4. A polypeptide according to Claim 1 or Claim 2 being unglycosylated.

5. A polypeptide according to any preceding claim in substantially pure form and essentially free from other mammalian hematopoietic cell proteins.

6. A process for producing a polypeptide as claimed in any of Claims 1 to 5, said process comprising the steps of:
(a) forming a vector comprising a nucleotide sequence coding for said polypeptide wherein the nucleotide sequence is capable of being expressed by a host containing the vector;
(b) incorporating the vector into the host;
and
(c) maintaining the host containing the vector under conditions suitable for expression of the nucleotide sequence into said polypeptide.

7. A process according to Claim 6 wherein the nucleotide sequence comprises the sequence

```
                                ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

8. A nucleic acid sequence that codes for a polypeptide as claimed in Claim 1 or Claim 2.

9. A nucleic acid sequence according to Claim 8 comprising the nucleotide sequence

```
                                ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

10. A vector consisting essentially of a DNA sequence of Claim 8 or Claim 9, especially when said vector is incorporated into a microorganism or cell.

17

**11.** A microorganism or cell transformed or transfected with the replicable expression vector of Claim 10.

**12.** A process for enhancing cell growth or differentiation comprising contacting the cell with a polypeptide as claimed in any of Claims 1 to 5.

**13.** A process for preparing a polypeptide exhibiting colony stimulating factor activity on human neutrophilic granulocytes, macrophages, and eosinophils, which comprises cultivating, in an aqueous nutrient medium, a prokaryotic microorganism or eukaryotic cell which has been transfected or transformed with a vector according to Claim 10.

**14.** A recombinant DNA molecule consisting of segments of DNA from different genomes which have been joined end to end outside of living cells and has the capacity to infect some host and to be maintained therein, and the progeny thereof said DNA sequence coding for a polypeptide as claimed in Claim 1 or Claim 2.

**15.** A recombinant DNA molecule according to Claim 14 comprising the DNA sequence

```
                                    ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

**16.** A pharmaceutical composition comprising a polypeptide as claimed in any of Claims 1 to 5 in association with a pharmaceutically acceptable carrier.

**Claims for the following Contracting State : AT**

**1.** A process for producing a recombinant polypeptide exhibiting colony stimulating factor activity on human neutrophilic granulocytes, macrophages, and eosinophils and having the structure

```
                                    Met Trp Leu Gln Ser Leu Leu Leu Leu
Gly Thr Val Ala Cys Ser Ile Ser Ala Pro Ala Arg Ser Pro Ser Pro Ser Thr
Gln Pro Trp Glu His Val Asn Ala Ile Gln Glu Ala Arg Arg Leu Leu Asn Leu
Ser Arg Asp Thr Ala Ala Glu Met Asn Glu Thr Val Glu Val Ile Ser Glu Met
Phe Asp Leu Gln Glu Pro Thr Cys Leu Gln Thr Arg Leu Glu Leu Tyr Lys Gln
Gly Leu Arg Gly Ser Leu Thr Lys Leu Lys Gly Pro Leu Thr Met Met Ala Ser
His Tyr Lys Gln His Cys Pro Pro Thr Pro Glu Thr Ser Cys Ala Thr Gln Ile
Ile Thr Phe Glu Ser Phe Lys Glu Asn Leu Lys Asp Phe Leu Leu Val Ile Pro
Phe Asp Cys Trp Glu Pro Val Gln Glu,
```

said process comprising the steps of:
    (a) forming a vector comprising a nucleotide sequence coding for said polypeptide wherein the

EP 0 202 300 B1

nucleotide sequence is capable of being expressed by a host containing the vector;
(b) incorporating the vector into the host;
and
(c) maintaining the host containing the vector under conditions suitable for expression of the nucleotide sequence into said polypeptide.

2. A process according to Claim 1 wherein said polypeptide is without a signal sequence.

3. A process according to Claim 1 or Claim 2 wherein said polypeptide is glycosylated.

4. A process according to Claim 1 or Claim 2 wherein said polypeptide is unglycosylated.

5. A process according to any preceding claim wherein said polypeptide is in substantially pure form and essentially free from other mammalian hematopoietic cell proteins.

6. A process according to any preceding claim wherein the nucleotide sequence comprises the sequence

```
                                    ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

7. A process for producing a nucleic acid sequence that codes for a polypeptide as defined in Claim 1 or Claim 2, which comprises culturing a host transformed with a recombinant plasmid or bacteriophage vector containing said nucleic acid sequence.

8. A process sequence according to Claim 7 wherein the nucleic acid sequence comprises nucleotide sequence

```
                                    ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

9. A vector consisting essentially of a DNA sequence as defined in Claim 7 or Claim 8, especially when said vector is incorporated into a microorganism or cell.

19

**10.** A microorganism or cell transformed or transfected with the replicable expression vector of Claim 9.

**11.** A process for enhancing cell growth or differentiation comprising contacting the cell with a polypeptide as defined in any of Claims 1 to 5.

**12.** A process for preparing a polypeptide exhibiting colony stimulating factor activity on human neutrophilic granulocytes, macrophages, and eosinophils, which comprises cultivating, in an aqueous nutrient medium, a prokaryotic microorganism or eukaryotic cell which has been transfected or transformed with a vector according to Claim 9.

**13.** A process for producing a recombinant DNA molecule consisting of segments of DNA from different genomes which comprises joining said segments end to end outside of living cells wherein said recombinant DNA molecule has the capacity to infect some host and to be maintained therein, and the progeny thereof, said DNA sequence coding for a polypeptide as claimed in Claim 1 or Claim 2.

**14.** A process according to Claim 13 wherein said recombinant DNA molecule comprises the DNA sequence

```
                                    ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

**15.** A pharmaceutical composition comprising a polypeptide as defined in any of Claims 1 to 5 in association with a pharmaceutically acceptable carrier.

**Patentansprüche**
**Patentansprüche für folgende Vertragsstaaten : BE, CH, DE, FR, GB, IT, LI, LU, NL, SE**

**1.** Rekombinantes Polypeptid mit der Wirksamkeit des kolonien-stimulierenden Faktors auf menschliche neutrophile Granulocyten, Makrophagen und Eosinophile und mit der Struktur

|     |     |     |     |     |     |     |     |     | Met | Trp | Leu |
| --- | --- | --- | --- | --- | --- | --- | --- | --- | --- | --- | --- |
| Gln | Ser | Leu | Leu | Leu | Leu | Gly | Thr | Val | Ala | Cys | Ser |
| Ile | Ser | Ala | Pro | Ala | Arg | Ser | Pro | Ser | Pro | Ser | Thr |
| Gln | Pro | Trp | Glu | His | Val | Asn | Ala | Ile | Gln | Glu | Ala |
| Arg | Arg | Leu | Leu | Asn | Leu | Ser | Arg | Asp | Thr | Ala | Ala |
| Glu | Met | Asn | Glu | Thr | Val | Glu | Val | Ile | Ser | Glu | Met |
| Phe | Asp | Leu | Gln | Glu | Pro | Thr | Cys | Leu | Gln | Thr | Arg |
| Leu | Glu | Leu | Tyr | Lys | Gln | Gly | Leu | Arg | Gly | Ser | Leu |
| Thr | Lys | Leu | Lys | Gly | Pro | Leu | Thr | Met | Met | Ala | Ser |
| His | Tyr | Lys | Gln | His | Cys | Pro | Pro | Thr | Pro | Glu | Thr |
| Ser | Cys | Ala | Thr | Gln | Ile | Ile | Thr | Phe | Glu | Ser | Phe |
| Lys | Glu | Asn | Leu | Lys | Asp | Phe | Leu | Leu | Val | Ile | Pro |
| Phe | Asp | Cys | Trp | Glu | Pro | Val | Gln | Glu |     |     |     |

2.  Polypeptid gemäß Anspruch 1 ohne eine Signalsequenz.

3.  Polypeptid gemäß Anspruch 1 oder 2, das glykosyliert ist.

4.  Polypeptid gemäß Anspruch 1 oder 2, das unglykosyliert ist.

5.  Polypeptid gemäß einem der vorhergehenden Ansprüche in im wesentlichen reiner Form und im wesentlichen frei von anderen Proteinen hämatopoetischer Säugerzellen.

6.  Verfahren zur Herstellung eines Polypeptids gemäß einem der Ansprüche 1 bis 5, mit den folgenden Schritten:
    (a) Bildung eines Vektors, der eine für dieses Polypeptid kodierende Nukleotidsequenz umfaßt, worin die Nukleotidsequenz die Fähigkeit zur Exprimierung in einem den Vektor enthaltenden Wirt aufweist;
    (b) Einbau des Vektors in den Wirt;
    und
    (c) Halten des den Vektors enthaltenden Wirts unter Bedingungen, die für die Expression der Nukleotidsequenz in dieses Polypeptid geeignet sind.

7.  Verfahren gemäß Anspruch 6, worin die Nukleotidsequenz die Sequenz

```
                                                    ATG   TGG   CTG
CAG  AGC  CTG  CTG  CTC  TTG  GGC  ACT  GTG  GCC   TGC   AGC
ATC  TCT  GCA  CCC  GCC  CGC  TCG  CCC  AGC  CCC   AGC   ACG
CAG  CCC  TGG  GAG  CAT  GTG  AAT  GCC  ATC  CAG   GAG   GCC
CGG  CGT  CTC  CTG  AAC  CTG  AGT  AGA  GAC  ACT   GCT   GCT
GAG  ATG  AAT  GAA  ACA  GTA  GAA  GTC  ATC  TCA   GAA   ATG
TTT  GAC  CTC  CAG  GAG  CCG  ACC  TGC  CTA  CAG   ACC   CGC
CTG  GAG  CTG  TAC  AAG  CAG  GGC  CTG  CGG  GGC   AGC   CTC
ACC  AAG  CTC  AAG  GGC  CCC  TTG  ACC  ATG  ATG   GCC   AGC
CAC  TAC  AAG  CAG  CAC  TGC  CCT  CCA  ACC  CCG   GAA   ACT
TCC  TGT  GCA  ACC  CAG  ATT  ATC  ACC  TTT  GAA   AGT   TTC
AAA  GAG  AAC  CTG  AAG  GAC  TTT  CTG  CTT  GTC   ATC   CCC
TTT  GAC  TGC  TGG  GAG  CCA  GTC  CAG  GAG
```

umfaßt.

8. Nukleinsäuresequenz, die für ein Polypeptid gemäß Anspruch 1 oder 2 kodiert.

9. Nukleinsäuresequenz gemäß Anspruch 8, umfassend die Nukleotidsequenz

```
                                                    ATG   TGG   CTG
CAG  AGC  CTG  CTG  CTC  TTG  GGC  ACT  GTG  GCC   TGC   AGC
ATC  TCT  GCA  CCC  GCC  CGC  TCG  CCC  AGC  CCC   AGC   ACG
CAG  CCC  TGG  GAG  CAT  GTG  AAT  GCC  ATC  CAG   GAG   GCC

CGG  CGT  CTC  CTG  AAC  CTG  AGT  AGA  GAC  ACT   GCT   GCT
GAG  ATG  AAT  GAA  ACA  GTA  GAA  GTC  ATC  TCA   GAA   ATG
TTT  GAC  CTC  CAG  GAG  CCG  ACC  TGC  CTA  CAG   ACC   CGC
CTG  GAG  CTG  TAC  AAG  CAG  GGC  CTG  CGG  GGC   AGC   CTC
ACC  AAG  CTC  AAG  GGC  CCC  TTG  ACC  ATG  ATG   GCC   AGC
CAC  TAC  AAG  CAG  CAC  TGC  CCT  CCA  ACC  CCG   GAA   ACT
TCC  TGT  GCA  ACC  CAG  ATT  ATC  ACC  TTT  GAA   ACT   TTC
AAA  GAG  AAC  CTG  AAG  GAC  TTT  CTG  CTT  GTC   ATC   CCC
TTT  GAC  TGC  TGG  GAG  CCA  GTC  CAG  GAG
```

10. Vektor, im wesentlichen aus einer DNA-Sequenz des Anspruchs 8 oder 9 bestehend, insbesondere wenn dieser Vektor in einen Mikroorganismus oder eine Zelle eingebaut ist.

11. Mikroorganismus oder Zelle, welche(r) mit dem replizierbaren Expressionsvektor des Anspruchs 10 transformiert oder transfiziert ist.

12. Verfahren zur Steigerung des Zellwachstums oder der Zelldifferenzierung, bei welchem die Zelle mit einem Polypeptid gemäß einem der Ansprüche 1 bis 5 in Kontakt gebracht wird.

13. Verfahren zur Herstellung eines Polypeptids mit der Wirksamkeit des kolonien-stimulierenden Faktors auf menschliche neutrophile Granulocyten, Makrophagen und Eosinophile, welches die Kultivierung eines prokaryotischen Mikroorganismus oder einer eukaryotischen Zelle, welche(r) mit einem Vektor gemäß Anspruch 10 transfiziert oder transformiert worden ist, in einem wäßrigen Nährmedium umfaßt.

14. Rekombinantes DNA-Molekül, bestehend aus DNA-Segmenten unterschiedlicher Genome, welche außerhalb lebender Zellen endseitig miteinander verknüpft worden sind und welche die Fähigkeit haben, einen Wirt zu infizieren und in diesem und dessen Nachkommen gehalten zu werden, wobei die vorerwähnte DNA-Sequenz für ein Polypeptid gemäß Anspruch 1 oder 2 kodiert.

15. Rekombinantes DNA-Molekül gemäß Anspruch 14, umfassend die DNA-Sequenz

|     |     |     |     |     |     |     |     |     | ATG | TGG | CTG |
|-----|-----|-----|-----|-----|-----|-----|-----|-----|-----|-----|-----|
| CAG | AGC | CTG | CTG | CTC | TTG | GGC | ACT | GTG | GCC | TGC | AGC |
| ATC | TCT | GCA | CCC | GCC | CGC | TCG | CCC | AGC | CCC | AGC | ACG |
| CAG | CCC | TGG | GAG | CAT | GTG | AAT | GCC | ATC | CAG | GAG | GCC |
| CGG | CGT | CTC | CTG | AAC | CTG | AGT | AGA | GAC | ACT | GCT | GCT |
| GAG | ATG | AAT | GAA | ACA | GTA | GAA | GTC | ATC | TCA | GAA | ATG |
| TTT | GAC | CTC | CAG | GAG | CCG | ACC | TGC | CTA | CAG | ACC | CGC |
| CTG | GAG | CTG | TAC | AAG | CAG | GGC | CTG | CGG | GGC | AGC | CTC |
| ACC | AAG | CTC | AAG | GGC | CCC | TTG | ACC | ATG | ATG | GCC | AGC |
| CAC | TAC | AAG | CAG | CAC | TGC | CCT | CCA | ACC | CCG | GAA | ACT |
| TCC | TGT | GCA | ACC | CAG | ATT | ATC | ACC | TTT | GAA | AGT | TTC |
| AAA | GAG | AAC | CTG | AAG | GAC | TTT | CTG | CTT | GTC | ATC | CCC |
| TTT | GAC | TGC | TGG | GAG | CCA | GTC | CAG | GAG |     |     |     |

16. Pharmazeutische Zubereitung umfassend ein Polypeptid gemäß einem der Ansprüche 1 bis 5 in Verbindung mit einem pharmazeutisch annehmbaren Träger.

**Patentansprüche für folgenden Vertragsstaat : AT**

1. Verfahren zur Herstellung eines rekombinanten Polypeptids mit der Wirksamkeit des kolonien-stimulierenden Faktors auf menschliche neutrophile Granulocyten, Makrophagen und Eosinophile und mit der Struktur

|     |     |     |     |     |     |     |     |     | Met | Trp | Leu |
| Gln | Ser | Leu | Leu | Leu | Leu | Gly | Thr | Val | Ala | Cys | Ser |
| Ile | Ser | Ala | Pro | Ala | Arg | Ser | Pro | Ser | Pro | Ser | Thr |
| Gln | Pro | Trp | Glu | His | Val | Asn | Ala | Ile | Gln | Glu | Ala |
| Arg | Arg | Leu | Leu | Asn | Leu | Ser | Arg | Asp | Thr | Ala | Ala |
| Glu | Met | Asn | Glu | Thr | Val | Glu | Val | Ile | Ser | Glu | Met |
| Phe | Asp | Leu | Gln | Glu | Pro | Thr | Cys | Leu | Gln | Thr | Arg |
| Leu | Glu | Leu | Tyr | Lys | Gln | Gly | Leu | Arg | Gly | Ser | Leu |
| Thr | Lys | Leu | Lys | Gly | Pro | Leu | Thr | Met | Met | Ala | Ser |
| His | Tyr | Lys | Gln | His | Cys | Pro | Pro | Thr | Pro | Glu | Thr |
| Ser | Cys | Ala | Thr | Gln | Ile | Ile | Thr | Phe | Glu | Ser | Phe |
| Lys | Glu | Asn | Leu | Lys | Asp | Phe | Leu | Leu | Val | Ile | Pro |
| Phe | Asp | Cys | Trp | Glu | Pro | Val | Gln | Glu, |     |     |     |

umfassend die Schritte:

(a) Bildung eines Vektors, der eine für dieses Polypeptid kodierende Nukleotidsequenz umfaßt, worin die Nukleotidsequenz die Fähigkeit zur Exprimierung in einem den Vektor enthaltenden Wirt aufweist;

(b) Einbau des Vektors in den Wirt; und

(c) Halten des den Vektors enthaltenden Wirts unter Bedingungen, die für die Expression der Nukleotidsequenz in dieses Polypeptid geeignet sind.

2. Verfahren gemäß Anspruch 1, worin das vorerwähnte Polypeptid ohne eine Signalsequenz ist.

3. Verfahren gemäß Anspruch 1 oder 2, worin das vorerwähnte Polypeptid glykosyliert ist.

4. Verfahren gemäß Anspruch 1 oder 2, worin das vorerwähnte Polypeptid unglykosyliert ist.

5. Verfahren gemäß einem der vorhergehenden Ansprüche, worin das vorerwähnte Polypeptid in im wesentlichen reiner Form und im wesentlichen frei ist von anderen Proteinen hämatopoetischer Säugerzellen.

6. Verfahren gemäß einem der vorhergehenden Ansprüche, worin die Nukleotidsequenz die Sequenz

|     |     |     |     |     |     |     |     |     | ATG | TGG | CTG |
|-----|-----|-----|-----|-----|-----|-----|-----|-----|-----|-----|-----|
| CAG | AGC | CTG | CTG | CTC | TTG | GGC | ACT | GTG | GCC | TGC | AGC |
| ATC | TCT | GCA | CCC | GCC | CGC | TCG | CCC | AGC | CCC | AGC | ACG |
| CAG | CCC | TGG | GAG | CAT | GTG | AAT | GCC | ATC | CAG | GAG | GCC |
| CGG | CGT | CTC | CTG | AAC | CTG | AGT | AGA | GAC | ACT | GCT | GCT |
| GAG | ATG | AAT | GAA | ACA | GTA | GAA | GTC | ATC | TCA | GAA | ATG |
| TTT | GAC | CTC | CAG | GAG | CCG | ACC | TGC | CTA | CAG | ACC | CGC |
| CTG | GAG | CTG | TAC | AAG | CAG | GGC | CTG | CGG | GGC | AGC | CTC |
| ACC | AAG | CTC | AAG | GGC | CCC | TTG | ACC | ATG | ATG | GCC | AGC |
| CAC | TAC | AAG | CAG | CAC | TGC | CCT | CCA | ACC | CCG | GAA | ACT |
| TCC | TGT | GCA | ACC | CAG | ATT | ATC | ACC | TTT | GAA | AGT | TTC |
| AAA | GAG | AAC | CTG | AAG | GAC | TTT | CTG | CTT | GTC | ATC | CCC |
| TTT | GAC | TGC | TGG | GAG | CCA | GTC | CAG | GAG |     |     |     |

umfaßt.

**7.** Verfahren zur Herstellung einer Nukleinsäuresequenz, die für ein wie in den Ansprüchen 1 oder 2 definiertes Polypeptid kodiert, umfassend die Züchtung eines mit einem rekombinanten Plasmid oder Bakteriophagenvektor transformierten Wirts, der die vorerwähnte Nukleinsäuresequenz enthält.

**8.** Verfahrenssequenz gemäß Anspruch 7, worin die Nukleinsäuresequenz die Nukleoidsequenz

|     |     |     |     |     |     |     |     |     | ATG | TGG | CTG |
|-----|-----|-----|-----|-----|-----|-----|-----|-----|-----|-----|-----|
| CAG | AGC | CTG | CTG | CTC | TTG | GGC | ACT | GTG | GCC | TGC | AGC |
| ATC | TCT | GCA | CCC | GCC | CGC | TCG | CCC | AGC | CCC | AGC | ACG |
| CAG | CCC | TGG | GAG | CAT | GTG | AAT | GCC | ATC | CAG | GAG | GCC |
| CGG | CGT | CTC | CTG | AAC | CTG | AGT | AGA | GAC | ACT | GCT | GCT |
| GAG | ATG | AAT | GAA | ACA | GTA | GAA | GTC | ATC | TCA | GAA | ATG |
| TTT | GAC | CTC | CAG | GAG | CCG | ACC | TGC | CTA | CAG | ACC | CGC |
| CTG | GAG | CTG | TAC | AAG | CAG | GGC | CTG | CGG | GGC | AGC | CTC |
| ACC | AAG | CTC | AAG | GGC | CCC | TTG | ACC | ATG | ATG | GCC | AGC |
| CAC | TAC | AAG | CAG | CAC | TGC | CCT | CCA | ACC | CCG | GAA | ACT |

|     |     |     |     |     |     |     |     |     |     |     |     |
|-----|-----|-----|-----|-----|-----|-----|-----|-----|-----|-----|-----|
| TCC | TGT | GCA | ACC | CAG | ATT | ATC | ACC | TTT | GAA | ACT | TTC |
| AAA | GAG | AAC | CTG | AAG | GAC | TTT | CTG | CTT | GTC | ATC | CCC |
| TTT | GAC | TGC | TGG | GAG | CCA | GTC | CAG | GAG |     |     |     |

umfaßt.

**9.** Vektor, im wesentlichen aus der in den Ansprüchen 7 oder 8 definierten DNA-Sequenz bestehend, insbesondere wenn dieser Vektor in einen Mikroorganismus oder eine Zelle eingebaut ist.

**10.** Mikroorganismus oder Zelle, welche(r) mit dem replizierbaren Expressionsvektor des Anspruchs 9 transformiert oder transfiziert ist.

**11.** Verfahren zur Steigerung des Zellwachstums oder der Zelldifferenzierung, bei welchem die Zelle mit einem Polypeptid gemäß einem der Ansprüche 1 bis 5 in Kontakt gebracht wird.

**12.** Verfahren zur Herstellung eines Polypeptids mit der Wirksamkeit des kolonien-stimulierenden Faktors auf menschliche neutrophile Granulocyten, Makrophagen und Eosinophile, welches die Kultivierung eines prokaryotischen Mikroorganismus oder einer eukaryotischen Zelle, welche(r) mit einem Vektor gemäß Anspruch 9 transfiziert oder transformiert worden ist, in einem wäßrigen Nährmedium umfaßt.

**13.** Verfahren zur Herstellung eines rekombinanten DNA-Moleküls, bestehend aus DNA-Segmenten unterschiedlicher Genome, welches die endseitige Verknüpfung der vorerwähnten Segmente miteinander außerhalb lebender Zellen umfaßt, worin die vorerwähnte rekombinanten DNA-Moleküle die Fähigkeit haben, einen Wirt zu infizieren und in diesem und dessen Nachkommen gehalten zu werden, und wobei die vorerwähnte DNA-Sequenz für ein Polypeptid gemäß Anspruch 1 oder 2 kodiert.

**14.** Verfahren gemäß Anspruch 13, worin das vorerwähnte rekombinante DNA-Molekül die DNA-Sequenz

```
                                              ATG  TGG  CTG
CAG  AGC  CTG  CTG  CTC  TTG  GGC  ACT  GTG  GCC  TGC  AGC
ATC  TCT  GCA  CCC  GCC  CGC  TCG  CCC  AGC  CCC  AGC  ACG
CAG  CCC  TGG  GAG  CAT  GTG  AAT  GCC  ATC  CAG  GAG  GCC
CGG  CGT  CTC  CTG  AAC  CTG  AGT  AGA  GAC  ACT  GCT  GCT
GAG  ATG  AAT  GAA  ACA  GTA  GAA  GTC  ATC  TCA  GAA  ATG
TTT  GAC  CTC  CAG  GAG  CCG  ACC  TGC  CTA  CAG  ACC  CGC
CTG  GAG  CTG  TAC  AAG  CAG  GGC  CTG  CGG  GGC  AGC  CTC
ACC  AAG  CTC  AAG  GGC  CCC  TTG  ACC  ATG  ATG  GCC  AGC
CAC  TAC  AAG  CAG  CAC  TGC  CCT  CCA  ACC  CCG  GAA  ACT
TCC  TGT  GCA  ACC  CAG  ATT  ATC  ACC  TTT  GAA  AGT  TTC
AAA  GAG  AAC  CTG  AAG  GAC  TTT  CTG  CTT  GTC  ATC  CCC
TTT  GAC  TGC  TGG  GAG  CCA  GTC  CAG  GAG
```

umfaßt.

**15.** Pharmazeutische Zubereitung umfassend ein wie in einem der Ansprüche 1 bis 5 definiertes Polypeptid in Verbindung mit einem pharmazeutisch annehmbaren Träger.

**Revendications**
**Revendications pour les Etats contractants suivants : BE, CH, DE, FR, GB, IT, LI, LU, NL, SE**

**1.** Polypeptide recombinant présentant une activité de facteur de stimulation de colonies sur les éosinophiles, macrophages et granulocytes neutrophiles humains et ayant la structure

```
                                        Met Trp Leu Gln Ser Leu Leu Leu Leu
Gly Thr Val Ala Cys Ser Ile Ser Ala Pro Ala Arg Ser Pro Ser Pro Ser Thr
Gln Pro Trp Glu His Val Asn Ala Ile Gln Glu Ala Arg Arg Leu Leu Asn Leu
Ser Arg Asp Thr Ala Ala Glu Met Asn Glu Thr Val Glu Val Ile Ser Glu Met
Phe Asp Leu Gln Glu Pro Thr Cys Leu Gln Thr Arg Leu Glu Leu Tyr Lys Gln
Gly Leu Arg Gly Ser Leu Thr Lys Leu Lys Gly Pro Leu Thr Met Met Ala Ser
His Tyr Lys Gln His Cys Pro Pro Thr Pro Glu Thr Ser Cys Ala Thr Gln Ile
Ile Thr Phe Glu Ser Phe Lys Glu Asn Leu Lys Asp Phe Leu Leu Val Ile Pro
Phe Asp Cys Trp Glu Pro Val Gln Glu
```

**2.** Polypeptide selon la revendication 1, sans séquence signal.

**3.** Polypeptide selon la revendication 1 ou la revendication 2, qui est glycosylé.

**4.** Polypeptide selon la revendication 1 ou la revendication 2, qui est non glycosylé.

**5.** Polypeptide selon toute revendication précédente sous forme sensiblement pure et essentiellement sans autres protéines de cellule hématopoïétique de mammifère.

**6.** Procédé de production d'un polypeptide selon l'une quelconque des revendications 1 à 5, ledit procédé comprenant les étapes de :
(a) former un vecteur comprenant une séquence de nucléotides codant pour ledit polypeptide où la séquence de nucléotides est capable d'être exprimée par un hôte contenant le vecteur ;
(b) incorporer le vecteur dans l'hôte ; et
(c) maintenir l'hôte contenant le vecteur dans des conditions appropriées à l'expression de la séquence de nucléotides dans ledit polypeptide.

**7.** Procédé selon la revendication 6, où la séquence de nucléotides comprend la séquence

```
                                        ATG TGG CTG CAG AGC CTG CTG CTC TTG
GGC ACT GTG GCC TGC AGC ATC TCT GCA CCC GCC CGC TCG CCC AGC CCC AGC ACG
GAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

**8.** Séquence d'acides nucléiques qui code pour un polypeptide selon la revendication 1 ou la revendication 2.

**9.** Séquence d'acides nucléiques selon la revendication 8 comprenant la séquence de nucléotides

```
                                      ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC ACC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

**10.** Vecteur consistant essentiellement en une séquence d'ADN de la revendication 8 ou de la revendication 9, en particulier quand ledit vecteur est incorporé dans un micro-organisme ou une cellule.

**11.** Micro-organisme ou cellule transformé ou transfecté par le vecteur d'expression réplicable de la revendication 10.

**12.** Procédé pour améliorer la croissance ou la différenciatior des cellules, comprenant la mise en contact de la cellule avec un polypeptide selon l'une quelconque des revendications 1 à 5.

**13.** Procédé de préparation d'un polypeptide présentant une activité de facteur de stimulation de colonies sur les éosinophiles, macrophages et granulocytes neutrophiles humains, qui comprend la mise en culture, dans un milieu nutritif aqueux, d'un micro-organisme procaryote ou d'une cellule eucaryote qui a été transfecté ou transformé avec un vecteur selon la revendication 10.

**14.** Molécule d'ADN recombinant consistant en segments d'ADN de différents génomes que l'on a joint bout à bout à l'extérieur des cellules vivantes et qui a la capacité d'infecter un hôte et de s'y maintenir, ainsi que sa descendance, ladite séquence d'ADN codant pour un polypeptide selon la revendication 1 ou la revendication 2.

**15.** Molécule d'ADN recombinant selon la revendication 14, comprenant la séquence d'ADN

```
                                      ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG CGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

**16.** Composition pharmaceutique comprenant un polypeptide selon l'une quelconque des revendications 1 à 5 en association avec un support acceptable en pharmacie.

**Revendications pour l'Etat contractant suivant : AT**

**1.** Procédé de production d'un polypeptide recombinant présentant une activité de facteur de stimulation de colonies sur les éosinophiles, macrophages et granulocytes neutrophiles humains et ayant la structure

```
                                    Met Trp Leu Gln Ser Leu Leu Leu Leu
Gly Thr Val Ala Cys Ser Ile Ser Ala Pro Ala Arg Ser Pro Ser Pro Ser Thr
Gln Pro Trp Glu His Val Asn Ala Ile Gln Glu Ala Arg Arg Leu Leu Asn Leu
Ser Arg Asp Thr Ala Ala Glu Met Asn Glu Thr Val Glu Val Ile Ser Glu Met
Phe Asp Leu Gln Glu Pro Thr Cys Leu Gln Thr Arg Leu Glu Leu Tyr Lys Gln
Gly Leu Arg Gly Ser Leu Thr Lys Leu Lys Gly Pro Leu Thr Met Met Ala Ser
His Tyr Lys Gln His Cys Pro Pro Thr Pro Glu Thr Ser Cys Ala Thr Gln Ile
Ile Thr Phe Glu Ser Phe Lys Glu Asn Leu Lys Asp Phe Leu Leu Val Ile Pro
Phe Asp Cys Trp Glu Pro Val Gln Glu,
```

ledit procédé comprenant les étapes de :
(a) former un vecteur comprenant une séquence de nucléotides codant pour ledit polypeptide où la séquence de nucléotides peut être exprimée par un hôte contenant le vecteur ;
(b) incorporer le vecteur dans l'hôte ;
et
(c) maintenir l'hôte contenant le vecteur dans des conditions appropriées à l'expression de la séquence de nucléotides dans ledit polypeptide.

**2.** Procédé selon la revendication 1 où ledit polypeptide est sans séquence signal.

**3.** Procédé selon la revendication 1 ou la revendication 2 où ledit polypeptide est glycosylé.

**4.** Procédé selon la revendication 1 ou la revendication 2 où ledit polypeptide est non glycosylé.

**5.** Procédé selon toute revendication précédente où ledit polypeptide est sous forme sensiblement pure et est essentiellement exempt d'autres protéines de cellule hématopoïétique de mammifère.

**6.** Procédé selon toute revendication précédente où la séquence de nucléotides comprend la séquence

```
                                    ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

**7.** Procédé de production d'une séquence d'acides nucléiques qui code pour un polypeptide tel que défini à la revendication 1 ou à la revendication 2, qui comprend la mise en culture d'un hôte transformé par un plasmide recombinant ou vecteur bactériophage contenant ladite séquence d'acides nucléiques.

**8.** Séquence de procédé selon la revendication 7 où la séquence d'acides nucléiques comprend la séquence de nucléotides

```
                                    ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TCC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

**9.** Vecteur consistant essentiellement en une séquence d'ADN telle que définie à la revendication 7 ou à la revendication 8, en particulier quand ledit vecteur est incorporé dans un micro-organisme ou une cellule.

**10.** Micro-organisme ou cellule transformé ou transfecté par le vecteur d'expression réplicable de la revendication 9.

**11.** Procédé pour améliorer la croissance des cellules ou leur différenciation, comprenant la mise en contact de la cellule avec un polypeptide tel que défini selon l'une des revendications 1 à 5.

**12.** Procédé de préparation d'un polypeptide présentant une activité de facteur de stimulation de colonies sur les éosinophiles, macrophages et granulocytes neutrophiles humains, qui comprend la mise en culture, dans un milieu nutritif aqueux, d'un micro-organisme procaryote ou d'une cellule eucaryote qui a été transfecté ou transformé par un vecteur selon la revendication 9.

**13.** Procédé de production d'une molécule d'ADN recombinant consistant en segments d'ADN de différents génomes qui comprend la jonction desdits segments bout à bout en dehors des cellules vivantes où ladite molécule d'ADN recombinant a la capacité d'infecter un hôte et de s'y maintenir ainsi que sa descendance, ladite séquence d'ADN codant pour un polypeptide selon la revendication 1 ou la revendication 2.

**14.** Procédé selon la revendication 13 où ladite molécule d'ADN recombinant comprend la séquence d'ADN

```
                                    ATG TGG CTG CAG AGC CTG CTG CTC TTG
GGC ACT GTG GCC TGC AGC ATC TCT GCA CCC GCC CGC TCG CCC AGC CCC AGC ACG
CAG CCC TGG GAG CAT GTG AAT GCC ATC CAG GAG GCC CGG CGT CTC CTG AAC CTG
AGT AGA GAC ACT GCT GCT GAG ATG AAT GAA ACA GTA GAA GTC ATC TCA GAA ATG
TTT GAC CTC CAG GAG CCG ACC TGC CTA CAG ACC CGC CTG GAG CTG TAC AAG CAG
GGC CTG CGG GGC AGC CTC ACC AAG CTC AAG GGC CCC TTG ACC ATG ATG GCC AGC
CAC TAC AAG CAG CAC TGC CCT CCA ACC CCG GAA ACT TTC TGT GCA ACC CAG ATT
ATC ACC TTT GAA AGT TTC AAA GAG AAC CTG AAG GAC TTT CTG CTT GTC ATC CCC
TTT GAC TGC TGG GAG CCA GTC CAG GAG
```

15. Composition pharmaceutique comprenant un polypeptide tel que défini selon l'une des revendications 1 à 5 en association avec un support acceptable en pharmacie.

FIGURE 1

HUMAN GM-CSF

```
          10         20        30                      47
ACACAGAGAG AAAGGCTAAA GTTCTCTGGA GG ATG TGG CTG CAG AGC CTG CTG CTC TTG
                                   MET Trp Leu Gln Ser Leu Leu Leu Leu

  62                 77                  92              107
GGC ACT GTG GCC TGC AGC ATC TCT GCA CCC GCC CGC TCG CCC AGC CCC AGC ACG
Gly Thr Val Ala Cys Ser Ile Ser Ala Pro Ala Arg Ser Pro Ser Pro Ser Thr

          122                137              152              167
CAG CCC TGG GAG CAT GTG AAT GCC ATC CAG GAG GCC CGG CGT CTC CTG AAC CTG
Gln Pro Trp Glu His Val Asn Ala Ile Gln Glu Ala Arg Arg Leu Leu Asn Leu

          182                197              212
AGT AGA GAC ACT GCT GCT GAG ATG AAT GAA ACA GTA GAA GTC ATC TCA GAA ATG
Ser Arg Asp Thr Ala Ala Glu Met Asn Glu Thr Val Glu Val Ile Ser Glu Met

  227                242                257              272
TTT GAC CTC CAG GAG CCG ACC TGC CTA CAG ACC CGC CTG GAG CTG TAC AAG CAG
Phe Asp Leu Gln Glu Pro Thr Cys Leu Gln Thr Arg Leu Glu Leu Tyr Lys Gln

          287                302              317
GGC CTG CGG GGC AGC CTC ACC AAG CTC AAG GGC CCC TTG ACC ATG ATG GCC AGC
Gly Leu Arg Gly Ser Leu Thr Lys Leu Lys Gly Pro Leu Thr Met Met Ala Ser

332                347                362              377
CAC TAC AAG CAG CAC TGC CCT CCA ACC CCG GAA ACT TCC TGT GCA ACC CAG ATT
His Tyr Lys Gln His Cys Pro Pro Thr Pro Glu Thr Ser Cys Ala Thr Gln Ile

          392                407              422              437
ATC ACC TTT GAA AGT TTC AAA GAG AAC CTG AAG GAC TTT CTG CTT GTC ATC CCC
Ile Thr Phe Glu Ser Phe Lys Glu Asn Leu Lys Asp Phe Leu Leu Val Ile Pro

          452                467       477       487       497
TTT GAC TGC TGG GAG CCA GTC CAG GAG TGA GACCGGCCAG ATGAGGCTGG CCAAGCCGGG
Phe Asp Cys Trp Glu Pro Val Gln Glu  .

     507        517        527        537        547        557        567
GAGCTGCTCT CTCATGAAAC AAGAGCTAGA AACTCAGGAT GGTCATCTTG GAGGGACCAA GGGGTGGGCC

     577        587        597        607        617        627        637
ACAGCCATGG TGGGAGTGGC CAGGACCTGC CCTGGGCACA CTGACCCTGA TACAGGCATG GCAGAAGAAT

     647        657        667        677        687        697        707
GGGAATATTT TATACTGACA GAAATCAGTA ATATTTATAT ATTTATATTT TTAAAATATT TATTTATTTA

     717        727        737        747        757        767        777
TTTATTTAAG TTCATATTCC ATATTTATTC AAGATGTTTT ACCGTAATAA TTATTATTAA AAATATGCTT

     787
CTAAAAAAAA
```

FIGURE 2

A.

pcD-HumGM-CSF

AmpR

PstI

pBR322 ori

Hind III

SV40ori early

splice junction

XhoI

PstI

G-C tail

PstI

BglI

cDNA insert

NcoI

A-T tail

XhoI

poly A

B.

G-C Tail

ApaI

A-T Tail

PstI

BglI

NcoI

AhaIII

100bp

FIGURE 3

COMPARISON OF HUMAN WITH MOUSE   GM-CSF

```
                                10
MET Trp Leu Gln Ser Leu Leu Leu Leu Gly Thr Val Ala Cys Ser Ile Ser Ala
                Asn         Phe      Ile     Val Tyr     Leu

    20                                  30
Pro Ala Arg Ser Pro Ser Pro Ser Thr Gln Pro Trp Glu His Val Asn Ala Ile
Thr             Ile Thr Val     Arg         Lys         Glu

        40                                      50
Gln Glu Ala Arg Arg Leu Leu Asn Leu Ser Arg Asp Thr Ala Ala Glu Met Asn
Lys         Leu Asn         Asp Asp Met Pro Val     Leu Asn     Glu Val

            *   60    *   *       *      *   *   *   70
Glu Thr Val Glu Val Ile Ser Glu Met Phe Asp Leu Gln Glu Pro Thr Cys Leu
    Val     |       Phe              |                              Val
    Ser|Asn                      Lys|Lys
                        80                                          90
Gln Thr Arg Leu Glu Leu Tyr Lys Gln Gly Leu Arg Gly Ser Leu Thr Lys Leu
        Lys Ile Phe Glu              Asn Phe

        `                   100
Lys Gly Pro Leu Thr Met Met Ala Ser His Tyr Lys Gln His Cys Pro Pro Thr
    Ala     Asn     Thr         Tyr     Gln Thr Tyr

    110                             120
Pro Glu Thr Ser Cys Ala Thr Gln Ile Ile Thr Phe Glu Ser Phe Lys Glu Asn
        Asp     Glu         Val Thr     Tyr Ala Asp     Ile Asp Ser

        130                             140
Leu Lys Asp Phe Leu Leu Val Ile Pro Phe Asp Cys Trp Glu Pro Val Gln Glu
    Thr         Thr Asp         Glu     Lys Lys     Ser     Lys
```

34